(19) Europäisches Patentamt
European Patent Office
Office européen des brevets

(11) EP 2 920 198 B1

(12) **EUROPEAN PATENT SPECIFICATION**

(45) Date of publication and mention of the grant of the patent:
**25.09.2019 Bulletin 2019/39**

(21) Application number: **13795420.2**

(22) Date of filing: **13.11.2013**

(51) Int Cl.:
***C07K 1/16*** $^{(2006.01)}$ ***C07K 1/18*** $^{(2006.01)}$
***C07K 1/20*** $^{(2006.01)}$

(86) International application number:
**PCT/DK2013/050378**

(87) International publication number:
**WO 2014/075688 (22.05.2014 Gazette 2014/21)**

(54) **PURIFICATION OF RECOMBINANT HUMAN GALACTOCEREBROSIDE BETA-GALACTOSIDASE (RHGALC)**

AUFREINIGUNG VON REKOMBINANTER MENSCHLICHER GALACTOCEREBROSID-BETA-GALAKTOSIDASE (RHGALC)

PURIFICATION DE GALACTOCÉREBROSIDE BÊTA-GALACTOSIDASE HUMAINE RECOMBINANTE (RHGALC)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.11.2012 DK 201270699**

(43) Date of publication of application:
**23.09.2015 Bulletin 2015/39**

(73) Proprietor: **Chiesi Farmaceutici S.p.A.**
**43122 Parma (IT)**

(72) Inventors:
- **FOGH, Jens**
  **3540 Lynge (DK)**
- **ANDERSSON, Claes**
  **187 43 Täby (SE)**
- **HYDÉN, Pia**
  **182 74 Stocksund (SE)**
- **GULSTAD, Pia Ringholm**
  **3400 Hillerød (DK)**
- **LUNDELL, Kerstin**
  **116 35 Stockholm (SE)**
- **HJERTMAN, Magnus**
  **118 28 Stockholm (SE)**

(74) Representative: **Plougmann Vingtoft a/s**
**Strandvejen 70**
**2900 Hellerup (DK)**

(56) References cited:
**WO-A2-2007/112757** **WO-A2-2008/073620**
**WO-A2-2011/163651**

- **YAMADA M ET AL: "Analysis of recombinant human saposin A expressed by Pichia pastoris", BIOCHEMICAL AND BIOPHYSICAL RESEARCH COMMUNICATIONS, ACADEMIC PRESS INC. ORLANDO, FL, US, vol. 318, no. 2, 28 May 2004 (2004-05-28), pages 588-593, XP004505420, ISSN: 0006-291X, DOI: 10.1016/J.BBRC.2004.04.069**
- **NAGANO SUKEHISA ET AL: "Expression and processing of recombinant human galactosylceramidase", CLINICA CHIMICA ACTA, vol. 276, no. 1, 10 August 1998 (1998-08-10), pages 53-61, XP002720380, ISSN: 0009-8981**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 920 198 B1

## Description

### Technical field of the invention

The present invention relates to a purification protocol of recombinant human galactocerebroside β-galactosidase and products obtainable by such process.

### Background of the invention

Galactocerebroside β-galactosidase is an enzyme that catalyzes the hydrolytic cleavage of galactose from galactocerebroside. Deficiency results in accumulation of galactocerebroside in tissues. Previous reported methods describe partial purification of human GALC from natural specimen, such as liver (Ben-Yoseph, Archives of Biochemistry and Biophysics, 1979), lymphocytes (Sakai et al, J. Biochem., 1994) and urine (Chen et al, Biochimica et Biophysica acta, 1993). The methods are complicated due to GALC's extreme hydrophobicity and low abundance. The recoveries are extremely low and the obtained products are described as mixtures of full length GALC (80 kDa) and processed forms (mainly 50 and 30 kDa). Furthermore, the aim with these previous reports on purifications was characterization of the enzyme and not production in large scale for enzyme replace therapy (ERT) in humans.

Hence, an improved purification protocol of rhGALC resulting in a homogenous product (80 kDa rhGALC) of high purity in a physiological solution suitable for human use would be advantageous.

### Summary of the invention

A process for purification of recombinant human Galactocerebroside β-Galactosidase (rhGALC) resulting in a final product fulfilling quality and purity requirements for animal/human studies has been developed. The process comprises three main chromatographic steps and optionally one UFDF formulation step. In this study fresh and clarified harvest from a 20 L fed batch bioreactor was purified with the optimized process to produce rhGALC for animal/human studies.

The purification protocol is based on the three-phase strategy consisting of capture, intermediate and polishing chromatographic steps with different modes of action. Preferably, all steps are performed in binding mode and include wash steps before elution. In an embodiment the capture step is Capto™ Blue, the intermediate step is Capto™ Adhere and the polishing step is Toyopearl Ether. Two dedicated virus inactivation/removal steps are included in embodiments of the process. The product pool may be formulated by UFDF prior to sterile filtration into the final product.

The final aim is to produce rhGALC as enzyme replacement therapy for treatment of the lysosomal enzyme storage disease Globoid Cell Leukodystrophy (Krabbe disease). Krabbe disease is caused by the genetic deficiency of the enzyme GALC. Deficiency of GALC results in the progressive accumulation of the sphingolipid metabolite galactosylsphingosine (psychosine), demyelination, and early death.

Thus, an object of the present invention relates to the provision of a purification protocol for rhGALC. In particular, it is an object of the present invention to provide a purification protocol for rhGALC that solves the above mentioned problems with usability in animals and humans.

Thus, one aspect of the invention relates to a process for purifying recombinant human Galactocerebroside β-Galactosidase (rhGALC) from a cell culture, wherein a fraction of said cell culture comprising rhGALC is subjected to chromatography on resins, the process comprising

a) A capture step in which said rhGALC is purified on a first multimodal chromatographic resin which binds through at least hydrophobic and electrostatic interactions and which comprises electrostatic ligands, optionially followed by virus inactivation and/or purification by ionic separation;

b) An intermediate step in which said rhGALC is purified on a second multimodal chromatographic resin comprising an anionic and hydrophobic ligand, optionially followed by virus inactivation and/or purification by ionic separation;

c) A polishing step in which said rhGALC is purified on a chromatographic resin which is selected from the group consisting of a multimodal chromatography resin, an anion exchange resin and a hydrophobic interaction chromatography (HIC) resin and wherein the third chromatographic resin comprises a ligand comprising an ether group.

In particular embodiments, the invention also provides a process for purifying recombinant human Galactocerebroside β-Galactosidase (rhGALC) from a cell culture, wherein a fraction of said cell culture comprising rhGALC is subjected to chromatography on resins, the process comprising

a) providing a fraction of said cell culture comprising rhGALC;

b) loading the fraction of said cell culture onto a first multimodal chromatographic resin which binds through at least hydrophobic and electrostatic interactions and which comprises electrostatic ligands;

c) eluting rhGALC from the first multimodal chromatographic resin in a first elution buffer comprising at least 30% propylene glycol and/or ethylene glycol (v/v) thereby providing a first eluate;

d) loading the first eluate onto a second multimodal chromatographic resin comprising an anionic and hydrophobic ligand;

e) eluting rhGALC from the second chromatographic resin in a second elution buffer comprising at least 30% propylene glycol and/or ethylene glycol (v/v) and having a pH below 5.5, thereby providing a second eluate;

f) loading the second eluate onto a third chromatographic resin having hydrophobic ligands; and

g) eluting rhGALC from the third chromatographic resin comprising a ligand comprising an ether group, which is selected from the group consisting of a multimodal chromatography resin, an anion exchange resin and a hydrophobic interaction chromatography (HIC) resin in an aqueous buffer, thereby providing a third eluate.

Another aspect of the present invention relates to a composition comprising rhGALC, wherein the molar ratio between full length rhGALC (80 kDa) and the main processed products (50 + 30 kDa) in said composition is at least 50:2.5. The composition may be one that is obtainable by the purification process according to the present invention.

Yet another aspect of the present invention is to provide the composition according to the present invention for use as a medicament.

Still another aspect of the present invention is to provide a composition according to the present invention for use in the treatment of Globoid Cell Leukodystrophy (Krabbe disease).

In yet a further aspect the invention relates to a purified rhGALC according to the present invention for use in a method of treating Globoid Cell Leukodystrophy (Krabbe disease) and/or reducing or alleviating the symptoms associated with Globoid Cell Leukodystrophy (Krabbe disease), said use comprising a step of administering a composition comprising a purified rhGALC according to the present invention to a subject in need thereof.

**Brief description of the figures**

*Figure 1*
Figure 1 shows an outline of the process for purification of rhGALC. Harvest from a 20 L bioreactor was purified in three pilot scale chromatographic cycles. The Ether products were pooled and the process continued in one cycle resulting in final product TG1106.

*Figure 2*
Figure 2 shows the yield (% activity) per step in the three chromatographic cycles as well as the UFDF and filtration cycles. Total yield from clarified harvest to final product is shown to the right. The activities were measured as replicates with at least two dilutions in two experiments per calculation, except for the Ether step in which the product was measured in one experiment.

*Figure 3*
Figure 3 shows a summary of total remaining activity from clarified harvest to final product. Activities were measured as in figure 2.

*Figure 4*
Figure 4 shows Western blot analysis of final product TG1106 and in-house standards StG02 and StG03. GALC was detected with a rabbit polyclonal antibody generated against StG02.

*Figure 5*
Figure 5 shows Western blot analysis of Ether products, UFDF product and final product TG1106 and in-house standard StG03 at excessive load. GALC was detected with a rabbit polyclonal antibody generated against StG02.

*Figure 6*

Figure 6 shows isoelectric focusing on pH 3-10 gels of TG1106 and in-house standard StG03.

*Figure 7*

Figure 7 shows Colloidal Blue stained SDS-PAGE of final product TG1106 and downstream in-process samples.

*Figures 8 and 9*

Figures 8 and 9 show Colloidal Blue stained SDS-PAGE of final product TG1106 and StG03 in various concentrations.

*Figure 10*

Figure 10 shows Colloidal Blue stained Native PAGE 4-16% Bis-Tris with G250 charge shift at neutral pH. Analysis of final product TG1106 and in-house standard StG03.

The present invention will now be described in more detail in the following.

**Detailed description of the invention**

**Process**

An aspect of the present disclosure provides a process for purifying recombinant human Galactocerebroside β-Galactosidase (rhGALC) from a cell culture, wherein a fraction of said cell culture comprising rhGALC is subjected to chromatography on resins, the process comprising

d) A capture step in which said rhGALC is purified on a first multimodal chromatographic resin, optionally followed by virus inactivation and/or purification by ionic separation;

e) An intermediate step in which said rhGALC is purified on a second multimodal chromatographic resin, optionally followed by virus inactivation and/or purification by ionic separation;

f) A polishing step in which said rhGALC is purified on a chromatographic resin which is selected from the group consisting of a multimodal chromatography resin, an anion exchange resin and a hydrophibic interaction chromatography (HIC) resin.

According to some embodiments of the invention, the said intermediate step comprises purification of said rhGALC on said second multimodal chromatographic resin, followed by purification of said rhGALC on a chromatography resin selected from the group consisting of:

i) a multimodal chromatography resin, which is different from said first and said second multimodal chromatographic resins; and
ii) a hydrophibic interaction chromatography (HIC) resin.

According to further embodiements of the invention, the said first and second multimodal chromatography resins are different resins.

The said first multimodal chromatographic resin may in particular comprise electrostatic ligands.

According to other embodiments of the invention, the said second multimodal chromatographic resin comprises an anionic and hydrophobic ligand.

In still further embodiments, the chromatographic resin in said polishing step is a resin having hydrophobic ligands.

The said first multimodal chromatographic resin may in particular comprises as ligand a compound of the formula (VI) as set forth hereinbelow.

The said second multimodal chromatographic resin may in particular comprise as ligand a compound of the formula (VIII) as set forth hereinbelow.

Accordng to further embodiments the said first multimodal chromatographic resin comprises as ligand a compound of the formula (VI) as set forth hereinbelow and the said second multimodal chromatographic resin comprises as ligand a compound of the formula (VIII) as set forth hereinbelow. According to these embodiments it is preferred that said rhGALC is subsequently purified on a chromatographic resin, which is an ether resin. Examples of suitable ether resins are given below.

According to some embodiments of the invention, the said rhGALC is eluted from said first multimodal chromatographic resin in first elution buffer comprising at least 30% propylene glycol and/or ethylene glycol (v/v).

In other embodiments of the invention, the said rhGALC is eluted from said second multimodal chromatographic resin in a second elution buffer comprising at least 30% propylene glycol and/or ethylene glycol (v/v) and having a pH below 5.5.

The process for purifying recombinant human Galactocerebroside β-Galactosidase (rhGALC) according to the disclosure may in particular comprise

a) providing a fraction of said cell culture comprising rhGALC;

b) loading the fraction of said cell culture onto a first multimodal chromatographic resin comprising eletrostatic ligands;

c) eluting rhGALC from the first multimodal chromatographic resin in a first elution buffer comprising at least 30% propylene glycol and/or ethylene glycol (v/v) thereby providing a first eluate;

d) loading the first eluate onto a second multimodal chromatographic resin comprising an anionic and hydrophobic ligand;

e) eluting rhGALC from the second chromatographic resin in a second elution buffer comprising at least 30% propylene glycol and/or ethylene glycol (v/v) and having a pH below 5.5, thereby providing a second eluate;

f) loading the second eluate onto a third chromatographic resin having hydrophobic ligands; and

g) eluting rhGALC from the third chromatographic resin in an aqueous s buffer, thereby providing a third eluate.

Several synonyms for Human Galactocerebroside β-Galactosidase (rhGALC) exist. The ones most commonly used are Galactocerebrosidase, Galactosylceramidase, Galcerase, Galactosylceramide beta-galactosidase (EC3.2.1.46). Protein accession number(s) are NP_000144.2 and P45803. It is to be understood that the rhGALC according to the present invention may comprise tags.

In the aspects and embodiments according to the invention the term Human Galactocerebroside β-Galactosidase (rhGALC) also includes functionally equivalent parts or analogues of the full length amino acid sequence.

Certain characteristics of rhGALC are important to know when setting up a purification protocol of rhGALC or functionally equivalent parts or analogues thereof.

- Approximately 80 kDa;
- 5 (or 6) glycosylation sites;
- Isoforms exists;
- pI theoterical 5.9, found 6.3;
- pH sensitive; pH 6.0-6.6 is preferred
- Extremely hydrophobic;
- Needs detergent to keep activity;
- Dimer, trimer, quatromer formation;

In the aspects and embodiments of the present disclosure as described herein, the rhGALC or said functionally equivalent part or analogue thereof comprises an amino acid sequence selected from the group consisting of:

i) an amino acid sequence as set forth in SEQ ID NO.: 2;
ii) a functionally equivalent part of an amino acid sequence as defined in i); and
iii) a functionally equivalent analogue of an amino acid sequence as defined in i) or ii), the amino acid sequence of said analogue being at least 75% identical to an amino acid sequence as defined in i) or ii).

In the context of the present invention the term "functionally equivalent" implies that the said part or analogue of rhGALC is able to hydrolyze the galactose ester bonds of galactocerebroside, galactosylsphingosine, lactosylceramide, monogalactosyldiglyceride, and the chromogenic substrate 2-hexadecanoylamino-4-nitrophenyl-b-D-galactopyranoside (HNG). In particular embodiments the said part or analogue of rhGALC retains at least 50%, such as at least 60%, at least 70%, at least 80%, at least 90% or at least 95% of the ability of the native enzyme (having the amino acid sequence set forth in SEQ ID NO: 2) to hydrolyse the galactose ester bonds of said compounds.

The catalytic properties of said part or analogue of rhGALC may be determined by measuring the hydrolysis of 2-hexadecanoylamino-4-nitrophenyl-b-D-galactopyranoside (HNG) into 2-hexadecanoylamino-4-nitrophenol (HN) at

pH 4.5. At pH 10.5, HN is a yellow colored product, which may be determined spectrophotometrically at 410 nm. An illustrative example of such measurements is provided in Example 12 in the present application.

In particular embodiments the analogue in iii) is at least 80% identical to a sequence as defined in i) or ii), such as at least 85%, at least 90%, at least 95%, at least 98%, at least 99%, or such as at least 99.5% identical to a sequence as defined in i) or ii).

Further, said rhGALC or said functionally equivalent part or analogue thereof may in particular be obtained by recombinant expression using a nucleic acid sequence comprising a sequence selected from the group consisting of:

i) a nucleic acid sequence as set forth in SEQ ID NO.: 1;
ii) a nucleic acid sequence which is at least 75% identical to a nucleic acid sequence as defined in i).

It may further be preferred that the acid sequence in ii) is at least 80% identical to a sequence as defined in i), such as at least 85%, at least 90%, at least 95%, at least 98%, at least 99%, or such as at least 99.5% identical to a sequence as defined in i).

The term "sequence identity" indicates a quantitative measure of the degree of homology between two amino acid sequences or between two nucleic acid sequences of equal or unequal length. If the two sequences to be compared are not of equal length, they must be aligned to give the best possible fit, allowing the insertion of gaps or, alternatively, truncation at the ends of the polypeptide sequences or nucleotide sequences. The sequence identity can be calculated as $\frac{(N_{ref} - N_{dif})100}{N_{ref}}$, wherein Ndif is the total number of non-identical residues in the two sequences when aligned and wherein Nref is the number of residues in one of the sequences. Hence, the DNA sequence AGTCAGTC will have a sequence identity of 75% with the sequence AATCAATC (Ndif=2 and Nref=8). A gap is counted as non-identity of the specific residue(s), i.e. the DNA sequence AGTGTC will have a sequence identity of 75% with the DNA sequence AGTCAGTC (Ndif=2 and Nref=8).

**Pre-resin steps**

The cell culture which provides the unpurfied rhGALC may come from different sources. In an embodiment the cell culture is provided from a bioreactor expressing rhGALC.

It may be an advantage to modify the fraction of the cell culture before applying to the first chromatographic resin. Thus, in an embodiment the pH of the fraction is adjusted to below 7 before loading onto the first chromatographic resin such as in the range 3-7, such as in the range 4-7, such as in the range 5-7 such as in the range 6-7, such as in the range 3-6, such as in the range 3-5, or such as in the range 3-4. It may also be advantageous to filter (e.g. depth, dead end or tangential flow filter) or centrifuge the cell culture to remove the cells before loading. Thus, in another embodiment the fraction of said cell culture is a filtered fraction. In yet an embodiment said fraction of the cell culture comprising rhGALC is a clarified undiluted harvest.

**First resin steps/capture steps**

The first resin stabilizes the enzyme and removes the media color. Different types of multimodalities may be suitable for the first multimodal chromatographic resin. Thus, in an embodiment the first multimodal chromatographic resin binds through at least hydrophobic and electrostatic interactions. In another embodiment the first multimodal chromatographic resin binds through at least aromatic and electrostatic interactions.

The said first multi modal chromatographic resin may comprise as ligand a compound of the formula (I), (II), (III), (V), (VI) or (VII) below.

In yet an embodiment the first multimodal chromatographic resin comprises as ligand a compound of the formula (I), (II) or (III) below.

In particular, the said first multi modal chromatographic resin may comprise as ligand a compound of the formula (V), (VI) or (VII).

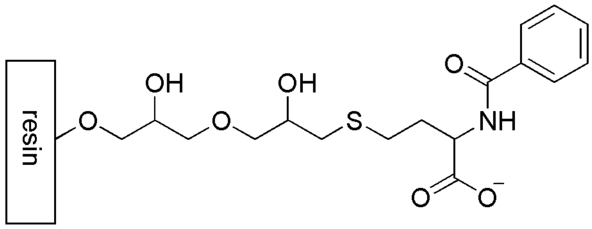

(I)

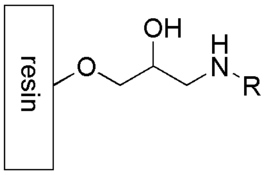

(II)

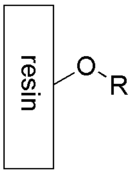

(III)

wherein R of the substances of formula (II) and (III) is a functional group of formula (IV):

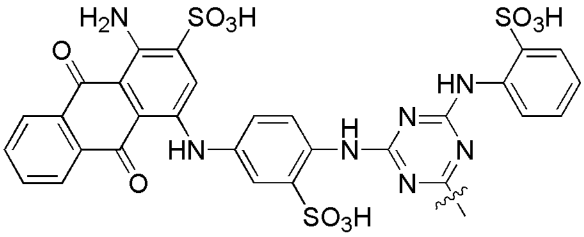

(IV)

(V)

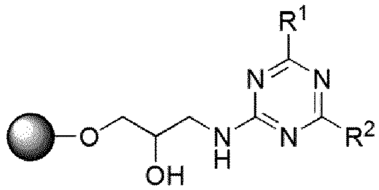

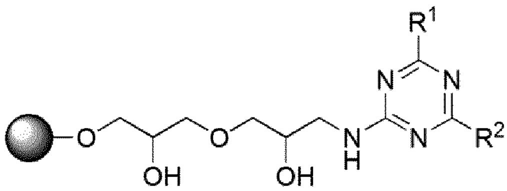

(VI)

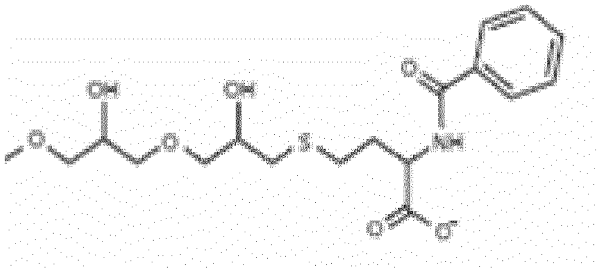
(VII)

In a further embodiment the first chromatographic resin comprises a ligand of the formula IV, formula V, formula VI or formula VII. Also more specific resins may be used as the first resin. Thus, in yet an embodiment the first resin is selected from the group consisting of "Capto™ MMC", "Capto™ Blue" (Capto™ Blue (high sub) and Capto™ Blue (low)), Capto™ Adhere, and "Blue sepharoseTM fast flow"; all available from GE Healthcare.

Capto MMC is a multimodal cation exchanger. It contains a carboxylic group and thus its features partly resemble those of a weak cation exchanger. However, in addition to the ionic interactions several other types of interactions are involved, including hydrogen bonding and hydrophobic interaction. Capto™ Blue is an affinity bioprocess media that binds through aromatic and electrostatic interactions. The Capto adhere ligand, N-Benzyl-N-methyl ethanolamine, exhibits many functionalities for interaction. The most pronounced are ionic interaction, hydrogen bonding and hydrophobic interaction.

In other embodiments the first resin is selected from the group consisting of MEP HyperCel™ Mixed-Mode Chromatography Sorbent, which is commercially available form Pall Corporation. MEP HyperCel sorbent operates by a mixed-mode or multi-mode mechanism also described as Hydrophobic Charge Induction Chromatography (HCIC). HCIC is based on the pH-dependent behavior of ionizable, dual-mode ligands.

In particular embodiments the first resin has a ligands of formula (VI). "Capto™ Blue" (Capto™ Blue (high sub) is an example of such a resin.

It is of course to be understood that the process according to the invention may also include some standard steps known to the skilled person. Thus, in an embodiment the first chromatographic resin is preconditioned before loading. In another embodiment after step b) the first chromatographic resin is washed in a wash buffer. In a further embodiment the first chromatographic resin is washed in a wash buffer comprising at the most 20% of propylene glycol and/or ethylene glycol (v/v), such as at the most 15%, such as at the most 10%, such as at the most 5%, such as in the range 5-20%, such as 5-15%, or such as around 10% propylene glycol and/or ethylene glycol. In an embodiment the wash buffer at the most 20% propylene glycol. In another embodiment the wash buffer at the most 20% ethylene glycol. Since rhGALC is extremely hydrophobic propylene glycol and/or ethylene glycol are preferred eluants.

The first elution buffer may have different components. In an embodiment wherein the first resin is "Capto™ Blue", the first elution buffer comprises a total concentration of propylene glycol and/or ethylene glycol (v/v) of 40-60%, such as 45-60%, such as 50-60%, such as 40-55% such as 40-50%, or such as around 50%. A high concentration of propylene glycol and/or ethylene glycol (v/v) is important for proper elution of the enzyme.

Following elution the buffer conditions may be changed. Thus, in an embodiment after step c) the total concentration of propylene glycol and/or ethylene glycol (v/v) in the first eluate is lowered to below 30% before step d), such as below 20%, such as below 15%, or such as below 10%. By lowering the concentration of propylene glycol and/or ethylene glycol the enzymatic stability of rhGALC is maintained. In a further embodiment after step c) the pH of the first eluate is adjusted to a pH in the range 5 to 6.5, such as 5.5 to 6.5 such as 5.7 to 6.3 or to around 6.1. Due to the pH sensitivity of rhGALC a pH in the range 6.0-6.6 is preferred. In yet a further embodiment, after step c) the level of detergent in the first eluate is adjusted to 0.01% to 5%, such as 0.5% to 5%, such as 0.5 to 4%, such as 0.5% to 3%, such as 0.5% to 2%, such as 0.5 to 1.5%. In another embodiment the detergent is a tween detergent such as tween 20, tween 40, tween 60 or tween 80. Tweens are also known as polysorbates The detergents should preferably be approved for human use, thus the detergent may e.g. also be Cremophor (Polyoxyl 35 castor oil) and Pluronic F-127.

In a further embodiment the first eluate is stored in the detergent for 5 hours to 48 hours, such as 10 hours to 3 hours, or such as 16 to 24 hours, e.g. at room temperature. By storing the first eluate for a longer period of time this step functions as a virus inactivation step, especially if the concentration of detergent is high such as 1%.

Before loading onto the second chromatographic resin, the first eluate may be preconditioned. Thus, in an embodiment, before step d) the first eluate is mixed with a preconditioning buffer for the second chromatographic resin. In yet an embodiment the mixture takes place by letting the first eluate enter directly into the preconditioning buffer for the second resin.

**Second resin steps/intermediate steps**

Different types of multimodalities may be suitable for the second multimodal chromatographic resin. Thus, in an embodiment the second chromatographic resin binds through ionic interactions, hydrogen binding and hydrophobic interactions. In another embodiment the second chromatographic resin comprises N-Benzyl-N-methyl ethanol amine as ligand. In a further embodiment the second chromatographic resin comprises a ligand of the formula:

[RESIN]- 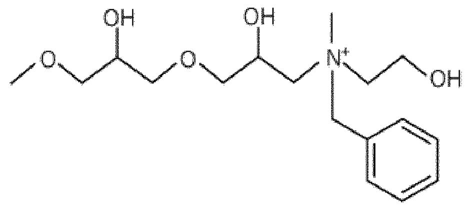 (VIII);

or a ligand of the formula:

[RESIN]- 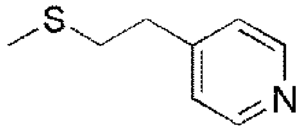 (IX)

In a more specific embodiment the second chromatographic resin is selected from the group consisting of Capto™ Adhere, CHT Ceramic Hydroxyapatite Type I (CHT I) which is commercially available from Biorad, and MEP HyperCel™ Mixed-Mode Chromatography Sorbent, which is commercially available form Pall Corporation.

Capto™ Adhere is a strong anion exchange bioprocess media with multimodal functionalities. It binds through ionic interactions, hydrogen binding and hydrophobic interaction. Ceramic Hydroxyapatite interacts with biomolecules by multiple modes: Cation exchange occurs when negatively charged phosphate groups interact with protein amino groups. Stronger coordination complexes can form between carboxyl clustes, phosphoryl moieties , or both, on biomolecules and the calcium sites on CHT ceramic hydroxyapatite via the mechanism of metal affinity. MEP HyperCel sorbent operates by a mixed-mode or multi-mode mechanism also described as Hydrophobic Charge Induction Chromatography (HCIC). HCIC is based on the pH-dependent behavior of ionizable, dual-mode ligands.

In particular embodiments the second resin comprises a ligand of the formula (VIII). Capto™ Adhere is an example of such a resin.

It is of course to be understood that the process according to the invention may also include some standard steps known to the skilled person. Thus, in an embodiment the second chromatographic resin is preconditioned before loading. In another embodiment, after step d), the second chromatographic resin is washed in a wash buffer. In a further embodiment, after step d) the second chromatographic resin is washed with a wash buffer with a pH in the range 3-5, such as 4-5, or such as 4.5-5. In yet a further embodiment the wash buffer further comprises an alcohol, such as isopropanol.

Different eluent buffers may be used for the second chromatographic resin. In an embodiment wherein the second chromatographic resin is Capto™ Adhere the second elution buffer comprises in the range 30-50% propylene glycol (v/v), such as 30-45%, such as 30-40%, such as 35-50%, such as 40-50%, or such as around 40%. As previously mentioned, since rhGALC is extremely hydrophobic propylene glycol and/or ethylene glycol are preferred eluents. Again rhGALC is also pH sensitive. Thus, in an embodiment the second elution buffer has a pH below 6 such as below 5, such as in the range 3-6, 4-6, or 4-5. In a further embodiment, before step f), the second eluate is mixed with a pre-conditioning buffer for the third chromatographic resin.

**Possible intermediate 2-step procedure**

In some embodiments according to the invention, the intermediate step is performed as a 2-step procedure as described in the following:

Intermediate step a)
Intermediate step a) in the 2-step procedure is performed essentially as described above; i.e. using a multi-modal resin and buffers as defined in connection with the second resin steps/intermediate steps.

Intermediate step b)

Different types of modalities may be suitable in intermediate step b), including multimodal resins, hydrophobic resins and chromatographic resins comprises a ligand with an ether group.

In a more particular embodiment the chromatographic resin used in intermediate step b) is selected from the group consisting of "PPG-600M", and "Toyopearl Phenyl-650M", which are commercially available from Tosoh Bioscience, "Capto™ Blue" (Capto™ Blue (high sub) and Capto™ Blue (low)), "Capto™ Butyl", Butyl-S Sepharose 6 (operated in bind-and-elute mode or in flow-through mode), and Macro-Prep Methyl HIC (operated in bind-and-elute mode or in flow-through mode) which is available from Biorad.

Toyopearl Phenyl-650M is a Hydrophobic Interaction Chromatography (HIC) medium. Capto™ Blue is an affinity bioprocess media that binds through aromatic and electrostatic interactions. "Capto™ Butyl" and Butyl-S Sepharose 6 are hydrophobic interaction chromatography (HIC) media. Macro-Prep methyl HIC support operates on a mechanism of interaction that is based on hydrophobicity and charge. The methyl groups are mildly hydrophobic. Depending on the pH of loading and elution buffers, the carboxyl groups can be exploited to ionically repel target molecules while the hydrophobic groups retain contaminants.

As the skilled person will realize, different eluent buffers may be used for the second chromatographic resin.

**Third resin steps/polishing steps**

Different types of modalities may be suitable for the third chromatographic resin. Thus, in an embodiment the third chromatographic resin comprises a ligand comprising an ether group. In another embodiment the third resin is hydrophobic. In yet an embodiment the third chromatographic resin comprises [resin]-$(OCH_2CH_2)_nOH$ as a ligand, wherein n is an integer in the range 1-20 such as 1-10, such as 1-5, such as 1-3, or such as 1-2.

In a more specific embodiment the third chromatographic resin is selected from the group consisting of ether resins, including Toyopearl Ether resins, such as 650M, 650S 5PW, "PPG-600M" and Toyopearl GigaCap Q-650, which are commercially available from Tosoh Bioscience,CHT Ceramic Hydroxyapatite Type I (CHT I) which is commercially available from Biorad, Q Sepharose Fast Flow (Q FF), which is commercially available from GE Healthcare, MEP HyperCel™ Mixed-Mode Chromatography Sorbent, which is commercially available form Pall Corporation, Macro-Prep Methyl HIC (operated in bind-and-elute mode or in flow-through mode), which is commercially available from BioRad, and Butyl-S Sepharose 6 (operated in bind-and-elute mode or in flow-through mode) and Capto DEAE, which are both commercially available from GE Healthcare.

Ceramic Hydroxyapatite interacts with biomolecules by multiple modes: Cation exchange occurs when negatively charged phosphate groups interact with protein amino groups. Much stronger coordination complexes can form between carboxyl clustes, phosphoryl moieties, or both, on biomolecules and the calcium sites on CHT ceramic hydroxyapatite via the mechanism of metal affinity

Q Sepharose Fast Flow is an ion exchange (IEX) chromatography medium (resin). MEP HyperCel sorbent operates by a mixed-mode or multi-mode mechanism also described as Hydrophobic Charge Induction Chromatography (HCIC). HCIC is based on the pH-dependent behavior of ionizable, dual-mode ligands. Toyopearl GigaCap Q-650 media is a high capacity, high resolution anion exchange resin, Macro-Prep methyl HIC support operates on a mechanism of interaction that is based on hydrophobicity and charge. Butyl-S Sepharose 6 is a hydrophobic interaction chromatography (HIC) medium.

In particular embodiments the third chromatographic resin is an ether resin, such as an ether resin selected from the group consisting of the said Toyopearl Ether resins, including 650M, 650S and 5PW. An advantage of this type of resin is that it does not require propylene/ethyleneglycol as eluent, but can use an aqueous eluent.

It is of course to be understood that the process according to the invention may also include some standard steps known to the skilled person. Thus, in an embodiment the third chromatographic resin is preconditioned before loading. Thus in an embodiment the preconditioning results in a buffer comprising 1-2 M NH4Ac and 1-2 M NH4Cl. In another embodiment, after step f) the third chromatographic resin is washed in a wash buffer. In yet an embodiment, after step f), the third chromatographic resin is washed with a wash buffer with a pH in the range 3-5, such as 4-5, such as 4.5-5, such as 5.5-7 or such as around 6.5. This is an advantage since rhGALC is pH sensitive.

Different wash buffers may be employed for the third resin. In an embodiment, after step f) the third chromatographic resin is washed with a first wash buffer comprising at least 1M NH4Ac, such as at least 2M or such as at least 3 M, or such as in the range 1-4M. In yet an embodiment the first wash buffer comprises at least 1M NH4Cl, such as at least 2M or such as at least 3 M, or such as in the range 1-3M and at least 0.1% detergent such as 0.1-2% detergent.

In an embodiment a second wash buffer contains lower salt and detergent concentrations than the first wash buffer. In yet an embodiment a third wash buffer contains lower salt concentrations than the second wash buffer. In a further embodiment the elution buffer is a sodium phosphate buffer.

For the purified rhGALC to be suitable for e.g. infusions into human the level of detergent should be low. Thus,

in an embodiment the third elution buffer comprises below 1% detergent, such as below 0.01%, such as below 0.001%, such as below 0.001% detergent. In yet an embodiment the detergent is a tween detergent, such as tween 80 or tween 20. In another embodiment the third elution buffer has a pH in the range 5-7, such as 6-7, or such as 6.2-6.8.

To improve the elution step the elution buffer may comprise salt. Thus, in a further embodiment the third elution buffer comprises at least 100 mM salt, such as NaCl and/or KCl. NaCl and KCl may be excluded, but the product may then be somewhat less pure.

In yet an embodiment the content of the final product is adjusted to comprise at least 150mM mannitol, such as at least 200 mM mannitol, such as at least 250, or such as in the range 200-400 mM mannitol, The presence of mannitol allows the product to be freeze-dried.

**Post resin steps**

To further minimize the levels of e.g. virus and other unwanted cell impurities further purification may be used. Thus, in an embodiment, after step g), the third eluate is passed to through a filter with a maximum filter size of 0.1 μm. In a further embodiment, after step g), the third eluate is passed to through a size exclusion filter with a filter size of at the most 20 nanometer, such as at the most 15 nanometer, such as a Planova 15N filter.

In yet an embodiment, the third eluate is further passed through a ultrafiltration/diafiltration step with tangential flow filtration (TFF) using a membrane with molecular weight cut off (MWCO) of below 50 kDa, such as below 30 kDa, such as below 15 kDa or such as below 10 kDa. In a further embodiment the membrane is a polyether sulfone membrane, such as a Pellicon polyether sulfone membrane. In yet an embodiment the membrane is a regenerated cellulose membrane.

**Additional steps**

In order to further improve product quality of the product according to the present invention it may be subjected to ionic separation. Optionally, the ionic separation may be applied between the capture step and the intermediate step, between the intermediate step and the polishing step or on the eluate from the polishing step

When performing ionic separation between the capture step and the intermediate step or after the polishing step, an anion filter or resin may be used, such as a Mustang® Q membrane, which is available from Pall Corporation. Mustang Q membranes are strong anion exchangers, which effectively bind plasmid DNA, negatively-charged proteins, and viral particles.

When performing ionic separation between intermediate step and the polishing step or after the plishing step it may be performed on a strong anion exchange (AIEX) resin, such as Capto Q, Giga Cap Q, Q FF. According to such embodiments, the resins are used in binding mode. It is also possible to include a weak anion exchange resin, such as Capto DEAE and DEAE FF, in particular when the polishing step uses hydrophobic interaction (HIC) chromatography.

In other embodiments the ionic separation may be applied immediately after the polishing step, such as immediately after elution from said ether resin.

In alternative embodiments the ionic separation may be applied after the ultrafiltration/diafiltration (UFDF) step.

Various different buffers may be used during the ionic separation. In particular embodiments, when applying ionic separation e.g. on a Mustang Q membrane immediately after the polishing step on ether resins, the anion filter or resin may be equilibrated with 3.7mM sodium phosphate, 5mM glycine, 10mM mannitol, 0.075 M NaCl 0.0005% tween 80, pH 6.2. The product may be diluted 1:1 (v:v) with 3.7mM sodium phosphate, 5mM glycine, 10mM mannitol, 0.0005% tween 80, pH 6.2 optionally with addition of 0.15 M NaCl, before it is run through the anion filter or resin.

In alternative embodiments, when applying ionic separation e.g. on a Mustang Q membrane after the UFDF step, the anion filter or resin may be equilibrated with 3.7mM sodium phosphate, 0.2 M NaCl, 5mM glycine, 10mM mannitol, 0.0005% tween 80, pH 6.2 The product may be diluted with 1M NaCl until conductivity is 20 mS/cm (approximately 1 volume product: 0.2 volume 1 M NaCl) before running it through the anion filter or resin.

Before sterile filtration the product may according to these embodiments be diluted with formulation buffer, such as a formulation buffer without NaCl to bring conductivity back to 15 mS/cm (0.15 M NaCl).

**Compositions**

The purified rhGALC according to the present invention differs from other purified rhGALC's, e.g. by purity, specific enzymatic activity, and the presence of processed products. Thus, in an aspect the invention relates to a composition comprising rhGALC.

In particular the rhGALC is one that is obtainable by the purification process according to the present invention.

Other purified products may comprise processed products of rhGALC. In an embodiment the molar ratio between full length rhGALC (80 kDa) and the main processed products (50+30 kDa) in the composition is at least 50:2.5, such

as at least 50:1, such as at least 100:1, such as at least 200:1, or such as 500:1. In another embodiment the ratio between full length rhGALC (80 kDa) and the two main processed products (30 kDa + 50 kDa) in the composition is at least 50:2.5, such as at least 100:1, such as at least 200:1, such as 500:1. The processed 30 and 50 kDa forms of rhGALC could be seen as minor bands and were estimated to <0.5% (see example section and figure 5).

In further embodiments, the composition according to the present invention contains very few host cell proteins. The skilled person will be aware of suitable methods for determining the content of host cell proteins and other contaminants. In particular, the level of host cell proteins may be determined by ELISA. Generally, the content of host cell proteins is satisfactory if it is 500 ng/mg or less. In some embodiments of the invention, the content of host cell proteins is 450 ng/mg or less, such as 300 ng/mg or less, or such as 250 ng/mg or less. In yet an embodiment the amount of host cell proteins is below 200 ng/mg in the composition, such as below 100 ng/mg rhGALC, such as below 40 ng/mg rhGALC or such as below 30 ng/mg rhGALC. As can be seen from the example section impurities were estimated to around 30 ng HCP per mg rhGALC by ELISA.

In some embodiments of the invention, the content of host cell proteins is 20 ng/mg or less.

In yet an embodiment the enzymatic activity in the composition is at least 15 kU/mL or such as at least 30 kU/mL. As can be seen from the example section the enzymatic activity in the final product was estimated to 42.5 kU/mL. The composition according to the present invention has, as one of its characteristics, a very high content of monomeric (80 kDa) rhGALC and a very low content of aggregates (dimers and multimers of rhGALC). Preferably, the amounts of aggregates are below the minimum level of detection, such as when detected by visual inspection. The composition according to the invention will then appear clear and not cloudy.

Alternatively, formation of aggregates and levels of aggregates may be measured by transmittance at 580 nm (T580). Using this methos a transmittance of >95% such as >96%, >96,5%, >97%, >98% or more than >99%, indicates satisfactory levels of aggregates. Another commonly used method is SEC (size exclusion chromatography).

The skilled person will be aware of other suitable methods for measuring/ assessing the level of protein aggregates, including dynamic light scattering and subvisual particles method (subvisual particle count).

In a preferred embodiment, less than 1.5% (w/w) of the rhGALC in said composition according to the invention is in the form of aggregates, such as less than 1% (w/w), e.g. less than 0.5% (w/w), less than 0.25%(w/w), less than 0.2% (w/w), less than 0.1% (w/w), less than 0.05% (w/w) or less than 0.01% (w/w). The content of monomeric (80 kDa) rhGALC s at least 95% (w/w), such as at least 96%(w/w), or at least 97% (w/w), e.g. at least 98% (w/w), preferably at least 98.5% ((w/w), at least 99.5% ((w/w), or 99% (w/w).

The compositions according to the present invention may find use as a medicament. Thus, an aspect of the present invention relates to the composition according to the present invention for use as a medicament.

In yet an aspect the invention relates to the composition according to the present invention for use in the treatment of Globoid Cell Leukodystrophy (Krabbe disease).

In a further aspect the invention relates to the use if the composition according to the present invention for the preparation of medicament for the treatment of Globoid Cell Leukodystrophy (Krabbe disease).

In yet a further aspect the invention relates to a purified rhGALC according to the present invention for use in a method of treating Globoid Cell Leukodystrophy (Krabbe disease) and/or reducing or alleviating the symptoms associated with Globoid Cell Leukodystrophy (Krabbe disease), said use comprising a step of administering a composition comprising a purified rhGALC according to the present invention to a subject in need thereof.

**Sequences**

SEQ ID NO.: 1

ggctactctc ggcttcctgg caacgccgag cgaaagctat gactgcggcc gcgggttcgg 60
cgggccgcgc cgcggtgccc ttgctgctgt gtgcgctgct ggcgcccggc ggcgcgtacg 120
tgctcgacga ctccgacggg ctgggccggg agttcgacgg catcggcgcg gtcagcggcg 180
gcggggcaac ctcccgactt ctagtaaatt acccagagcc ctatcgttct cagatattgg 240
attatctctt taagccgaat tttggtgcct ctttgcatat tttaaaagtg gaaataggtg 300
gtgatgggca gacaacagac ggcactgagc cctcccacat gcattatgca ctagatgaga 360
attatttccg aggatacgag tggtggttga tgaaagaagc taagaagagg aatcccaata 420
ttacactcat tgggttgcca tggtcattcc ctggatggct gggaaaaggt ttcgactggc 480
cttatgtcaa tcttcagctg actgcctatt atgtcgtgac ctggattgtg ggcgccaagc 540
gttaccatga tttggacatt gattatattg gaatttggaa tgagaggtca tataatgcca 600
attatattaa gatattaaga aaaatgctga attatcaagg tctccagcga gtgaaaatca 660
tagcaagtga taatctctgg gagtccatct ctgcatccat gctccttgat gccgaactct 720
tcaaggtggt tgatgttata ggggctcatt atcctggaac ccattcagca aaagatgcaa 780
agttgactgg gaagaagctt tggtcttctg aagactttag cactttaaat agtgacatgg 840
gtgcaggctg ctggggtcgc attttaaatc agaattatat caatggctat atgacttcca 900
caatcgcatg gaatttagtg gctagttact atgaacagtt gccttatggg agatgcgggt 960

tgatgacggc ccaagagcca tggagtgggc actacgtggt agaatctcct gtctgggtat 1020

cagctcatac cactcagttt actcaacctg gctggtatta cctgaagaca gttggccatt 1080
tagagaaagg aggaagctac gtagctctga ctgatggctt agggaacctc accatcatca 1140
ttgaaaccat gagtcataaa cattctaagt gcatacggcc atttcttcct tatttcaatg 1200
tgtcacaaca atttgccacc tttgttctta agggatcttt tagtgaaata ccagagctac 1260
aggtatggta taccaaactt ggaaaaacat ccgaaagatt tctttttaag cagctggatt 1320
ctctatggct ccttgacagc gatggcagtt tcacactgag cctgcatgaa gatgagctgt 1380
tcacactcac cactctcacc actggtcgca aaggcagcta cccgcttcct ccaaaatccc 1440
agcccttccc aagtacctat aaggatgatt tcaatgttga ttacccattt tttagtgaag 1500
ctccaaactt tgctgatcaa actggtgtat ttgaatattt tacaaatatt gaagaccctg 1560
gcgagcatca cttcacgcta cgccaagttc tcaaccagag acccattacg tgggctgccg 1620
atgcatccaa cacaatcagt attataggag actacaactg gaccaatctg actataaagt 1680
gtgatgttta catagagacc cctgacacag gaggtgtgtt cattgcagga agagtaaata 1740
aaggtggtat tttgattaga agtgccagag gaattttctt ctggattttt gcaaatggat 1800
cttacagggt tacaggtgat ttagctggat ggattatata tgctttagga cgtgttgaag 1860
ttacagcaaa aaaatggtat acactcacgt taactattaa gggtcatttc gcctctggca 1920
tgctgaatga caagtctctg tggacagaca tccctgtgaa ttttccaaag aatggctggg 1980
ctgcaattgg aactcactcc tttgaatttg cacagtttga caactttctt gtggaagcca 2040
cacgctaata cttaacaggg catcatagaa tactctggat tttcttccct tctttttggt 2100
tttggttcag agccaattct tgtttcattg gaacagtata tgaggctttt gagactaaaa 2160
ataatgaaga gtaaaagggg agagaaattt atttttaatt taccctgtgg aagattttat 2220
tagaattaat tccaagggga aaactggtga atctttaaca ttacctggtg tgttccctaa 2280
cattcaaact gtgcattggc cataccctta ggagtggttt gagtagtaca gacctcgaag 2340
ccttgctgct aacactgagg tagctctctt catcttattt gcaagcggtc ctgtagatgg 2400
cagtaacttg atcatcactg agatgtattt atgcatgctg accgtgtgtc caagtgagcc 2460
agtgtcttca tcacaagatg atgctgccat aatagaaagc tgaagaacac tagaagtagc 2520
tttttgaaaa ccacttcaac ctgttatgct ttatgctcta aaaagtattt ttttattttc 2580
ctttttaaga tgatactttt gaaatgcagg atatgatgag tgggatgatt ttaaaaacgc 2640
ctctttaata aactacctct aacactattt ctgcggtaat agatattagc agattaattg 2700
ggttatttgc attatttaat ttttttgatt ccaagttttg gtcttgtaac cactataact 2760
ctctgtgaac gtttttccag gtggctggaa gaaggaagaa aacctgatat agccaatgct 2820
gttgtagtcg tttcctcagc ctcatctcac tgtgctgtgg tctgtcctca catgtgcact 2880
ggtaacagac tcacacagct gatgaatgct tttctctcct tatgtgtgga aggaggggag 2940
cacttagaca tttgctaact cccagaattg gatcatctcc taagatgtac ttacttttta 3000

aagtccaaat atgtttatat ttaaatatac gtgagcatgt tcatcatgtt gtatgattta 3060

tactaagcat taatgtggct ctatgtagca aatcagttat tcatgtaggt aaagtaaatc 3120
tagaattatt tataagaatt actcattgaa ctaattctac tatttaggaa tttataagag 3180
tctaacatag gcttagctac agtgaagttt tgcattgctt ttgaagacaa gaaaagtgct 3240
agaataaata agattacaga gaaaattttt tgttaaaacc aagtgatttc cagctgatgt 3300
atctaatatt ttttaaaaca aacattatag aggtgtaatt tatttacaat aaaatgttcc 3360
tactttaaat atacaattca gtgagttttg ataaattgat atacccatgt aaccaacact 3420
ccagtcaagc ttcagaatat ttccatcacc ccagaaggtt ctcttgtata cctgctcagt 3480
cagttccttt cactcccaat tgttggcagc cattgatagg aattctatca ctataggtta 3540
gttttctttg ttccagaaca tcatgaaagc ggcgtcatgt actgtgtatt cttatgaatg 3600
gtttctttcc atcagcataa tgatttgaga ttggtccatg ttgtgtgatt cagtggtttg 3660
ttccttctta tttctgaaga gttttccatt gtatgaatat accacaattt gtttcctccc 3720
caccagtttc tgatactaca attaaaactg tctacattta c 3761

SEQ ID NO.: 2

Met Thr Ala Ala Ala Gly Ser Ala Gly Arg Ala Ala Val Pro Leu Leu
Leu Cys Ala Leu Leu Ala Pro Gly Gly Ala Tyr Val Leu Asp Asp Ser
Asp Gly Leu Gly Arg Glu Phe Asp Gly Ile Gly Ala Val Ser Gly Gly
Gly Ala Thr Ser Arg Leu Leu Val Asn Tyr Pro Glu Pro Tyr Arg Ser
Gln Ile Leu Asp Tyr Leu Phe Lys Pro Asn Phe Gly Ala Ser Leu His
Ile Leu Lys Val Glu Ile Gly Gly Asp Gly Gln Thr Thr Asp Gly Thr
Glu Pro Ser His Met His Tyr Ala Leu Asp Glu Asn Tyr Phe Arg Gly
Tyr Glu Trp Trp Leu Met Lys Glu Ala Lys Lys Arg Asn Pro Asn Ile
Thr Leu Ile Gly Leu Pro Trp Ser Phe Pro Gly Trp Leu Gly Lys Gly
Phe Asp Trp Pro Tyr Val Asn Leu Gln Leu Thr Ala Tyr Tyr Val Val
Thr Trp Ile Val Gly Ala Lys Arg Tyr His Asp Leu Asp Ile Asp Tyr
Ile Gly Ile Trp Asn Glu Arg Ser Tyr Asn Ala Asn Tyr Ile Lys Ile
Leu Arg Lys Met Leu Asn Tyr Gln Gly Leu Gln Arg Val Lys Ile Ile
Ala Ser Asp Asn Leu Trp Glu Ser Ile Ser Ala Ser Met Leu Leu Asp
Ala Glu Leu Phe Lys Val Val Asp Val Ile Gly Ala His Tyr Pro Gly
Thr His Ser Ala Lys Asp Ala Lys Leu Thr Gly Lys Lys Leu Trp Ser
Ser Glu Asp Phe Ser Thr Leu Asn Ser Asp Met Gly Ala Gly Cys Trp
Gly Arg Ile Leu Asn Gln Asn Tyr Ile Asn Gly Tyr Met Thr Ser Thr
Ile Ala Trp Asn Leu Val Ala Ser Tyr Tyr Glu Gln Leu Pro Tyr Gly
Arg Cys Gly Leu Met Thr Ala Gln Glu Pro Trp Ser Gly His Tyr Val
Val Glu Ser Pro Val Trp Val Ser Ala His Thr Thr Gln Phe Thr Gln

Pro Gly Trp Tyr Tyr Leu Lys Thr Val Gly His Leu Glu Lys Gly Gly
Ser Tyr Val Ala Leu Thr Asp Gly Leu Gly Asn Leu Thr Ile Ile Ile
Glu Thr Met Ser His Lys His Ser Lys Cys Ile Arg Pro Phe Leu Pro
Tyr Phe Asn Val Ser Gln Gln Phe Ala Thr Phe Val Leu Lys Gly Ser
Phe Ser Glu Ile Pro Glu Leu Gln Val Trp Tyr Thr Lys Leu Gly Lys
Thr Ser Glu Arg Phe Leu Phe Lys Gln Leu Asp Ser Leu Trp Leu Leu
Asp Ser Asp Gly Ser Phe Thr Leu Ser Leu His Glu Asp Glu Leu Phe
Thr Leu Thr Thr Leu Thr Thr Gly Arg Lys Gly Ser Tyr Pro Leu Pro
Pro Lys Ser Gln Pro Phe Pro Ser Thr Tyr Lys Asp Asp Phe Asn Val
Asp Tyr Pro Phe Phe Ser Glu Ala Pro Asn Phe Ala Asp Gln Thr Gly
Val Phe Glu Tyr Phe Thr Asn Ile Glu Asp Pro Gly Glu His His Phe
Thr Leu Arg Gln Val Leu Asn Gln Arg Pro Ile Thr Trp Ala Ala Asp
Ala Ser Asn Thr Ile Ser Ile Ile Gly Asp Tyr Asn Trp Thr Asn Leu
Thr Ile Lys Cys Asp Val Tyr Ile Glu Thr Pro Asp Thr Gly Gly Val
Phe Ile Ala Gly Arg Val Asn Lys Gly Gly Ile Leu Ile Arg Ser Ala
Arg Gly Ile Phe Phe Trp Ile Phe Ala Asn Gly Ser Tyr Arg Val Thr
Gly Asp Leu Ala Gly Trp Ile Ile Tyr Ala Leu Gly Arg Val Glu Val
Thr Ala Lys Lys Trp Tyr Thr Leu Thr Leu Thr Ile Lys Gly His Phe
Ala Ser Gly Met Leu Asn Asp Lys Ser Leu Trp Thr Asp Ile Pro Val
Asn Phe Pro Lys Asn Gly Trp Ala Ala Ile Gly Thr His Ser Phe Glu
Phe Ala Gln Phe Asp Asn Phe Leu Val Glu Ala Thr Arg

It should be noted that embodiments and features described in the context of one of the aspects of the present invention also apply to the other aspects of the invention.

The invention will now be described in further details in the following non-limiting examples.

**Examples**

A downstream process (DSP) for purification of recombinant human Galactocerebroside β-Galactosidase (rh-GALC) resulting in a final product fulfilling quality and purity requirements for animal studies was developed and tested in pilot scale. The process consists of three chromatographic steps and one UFDF formulation step. In this study fresh and clarified harvest from a 20 L fed batch bioreactor was purified with an optimized process in pilot scale to produce rhGALC for animal studies and e.g. human treatment protocols. The protocol is summarized in figure 1.

**Example 1**

Equipment, materials, buffers and methods

Equipment

Chromatographic system:

- Biological Duo-Flow upgraded with a Maximizer 80 (BioRad).

Peristaltic pump:

- MasterFlex L/S model 77200-60 (Cole-Parker Instrument Company) Tangential flow filtration system:
- Pellicon 2 Mini filter holder with manometers (Millipore) and peristaltic pump Watson Marlow SciQ 323, tubing with 6 mm IØ.

Magnetic stirrer:

- MR 3001 k (Heidolph)

Scales:

- EA35EDE-I maximum 35 kg (Sartorius)
- BP1200 maximum 1200 g (Sartorius)

Columns:

- Index 70/500 (GE Healthcare)

LAF bench:

- LaminarAir (Holten)

<u>Resins, filters and containers</u>

Harvest filter:

- Millistak+® Pod C0HC 0.054 $m^2$ (Millipore)

Chromatographic resins:

Capture step:

- Capto™ Blue (high sub) (GE Healthcare)

Intermediate step:

- Capto™ Adhere (GE Healthcare)

Polishing step:

- Toyopearl Ether-650M (Tosoh)

UFDF cassette:

- Pellicon 2 MINI 30 K (30 kDa MWCO, V-screen, 0.1 $m^2$, cat. no P2B030V01, Millipore)

Sterile filtration:

- 0.22 μm PES, 75 mm diameter, (NALGENE cat. no 595-4520)

Containers for final product:

- Sterile 30 mL bottles (Nalgene)
- Sterile 1.8 mL cryogenic vials (Nalgene)

Buffers

Buffers were prepared from chemicals of p.a. quality and water of Milli-Q quality. The buffers were filtered through 0.22 μm and stored at room temperature for maximum 5 days. Preparation recipes are shown in tables 1-4 for respective step.

Capto™ Blue buffers:

a) Conditioning: 40 mM sodium phosphate, pH 6.1±0.1
b) Equilibration: 20 mM sodium phosphate, 0.1% tween 80 (t-80) (w:w), 5% glycerol volume:volume (v:v), pH 6.1±0.1
c) Wash: 100 mM sodium phosphate, 1.5 M NaCl, 10% propylene glycol (1,2-propaneidole) (v:v), 5% isopropanol (IPA) (v:v), 0.2% t-80 (w:w), pH 7.0 ±0.1
d) Wash 2: Equilibration buffer
e) Elution buffer: 20 mM sodium phosphate, 50% propylene glycol (v:v), 0.5% t-80, 1 M NaCl, pH 6.5±0.1
f) Elution mix: 20 mM sodium phosphate, 0.15 M NaCl, 1.3% t-80, pH 6.1±0.1

Table 1: Buffer preparation per L for the capture step: Capto Blue (high sub)

| Buffer | Condition | Equilibration | Wash | Elution | Elution mix |
|---|---|---|---|---|---|
| Volume | 1 L | 1 L | 1 L | 1 L | 1 L |
| $Na_2HPO_4$ x 2 $H_2O$ 178 g/mol (g) | 0.93 | 0.42 | 15.1 | 1.6 | 0.71 |
| $NaH_2PO_4$ x 2 $H_2O$ 156 g/mol (g) | 5.43 | 2.75 | 2.3 | 1.72 | 2.5 |
| NaCl (g) | | | 87.6 | 58.4 | 8.8 |
| Tween 80 (g) | | 1 | 2 | 5 | 13 |
| Glycerol (g) | | 62.5 | | | |
| Isopropanol (g) | | | 39.3 | | |
| $H_2O$ (g) | 996 | 941 | 818 | 481 | 984 |
| Propylene glycol (g) | | | 104 | 521 | |
| pH | 6.1±0.1 | 6.1±0.1 | 7.0±0.1 | 6.5±0.1 | 6.1±0.1 |

(continued)

| Buffer | Condition | Equilibration | Wash | Elution | Elution mix |
|---|---|---|---|---|---|
| Conductivity (mS/cm) | | 160±15 | 75±10 | 17±3 | |

Capto™ Adhere buffers:

a) Equilibration buffer: 20 mM sodium phosphate, 0.15 M NaCl, 0.05% t-80 (w.w)), pH 6.1±0.1

b) Wash 1 buffer: 200 mM sodium acetate, 1 M NaCl, 5% IPA (v:v), 0.5% t-80 (w:w), pH 4.7±0.1

c) Wash 2 buffer: 10 mM sodium acetate, 0.1% t-80 (w:w), pH 4.7±0.1

d) Wash 3 buffer: 50% wash 2 and 50% elution buffer

e) Elution buffer: 10 mM sodium acetate, 300 mM NaCl, 0.1% t-80 (w:w), 5% IPA (v:v), 40% propylene glycol (v:v), pH 4.55±0.1

f) Elution mix buffer: 140 mM sodium phosphate, 0.0005% t-80 (w:w), pH 6.5±0.2

Table 2: Buffer preparation per L for the intermediate step: Capto Blue Adhere

| Buffer | Equilibration | Wash 1 | Wash 2 | Elution | Elution mix |
|---|---|---|---|---|---|
| Volume | 1 L | 1 L | 1 L | 1 L | 1 L |
| $Na_2HPO_4 \times 2\,H_2O$ 178 g/mol (g) | 0.71 | | | | 8.7 |
| $NaH_2PO_4 \times 2\,H_2O$ 156 g/mol (g) | 2.5 | | | | 14.2 |
| NaCl (g) | 8.8 | 58.4 | | 17.53 | |
| $C_2H_3NaO_2$ (82 g/mol) (g) | | 16.4 | 0.82 | 0.82 | |
| Tween 80 (g) | 0.5 | 5 | 1 | 1 | 1 g of 0.5% stock |
| Isopropanol (g) | | 39 | | 39 | |
| $H_2O$ (g) | 984 | 921 | 996 | 607 | 989 |
| Glacial acetic acid (g) | | 6.4 | 0.4 | 1.9 | |
| Propylene glycol (g) | | | | 415 | |
| pH | 6.1±0.1 | 4.7±0.1 | 4.7±0.1 | 4.55±0.1 | 6.5±0.2 |
| Conductivity (mS/cm) | | 75±10 | 1.5±0.5 | 8+2 | 12±2 |

Toyopearl Ether-650M buffers:

a) Conditioning buffer: 3.3 M $NH_4Ac$, 2.6 M $NH_4Cl$, 0.1% t-80 (w:w), pH 6.1±0.1

b) Equilibration buffer: 1.6 M $NH_4Ac$, 1.2 M $NH_4Cl$, 50 mM sodium phosphate, 0.0005% t-80 (w:w),, pH 6.4±0.1

c) Wash 1: 3.3 M $NH_4Ac$, 2.3 M $NH_4Cl$, 0.5% t-80 (w:w), pH 6.5

d) Wash 2: Equilibration buffer

e) Wash 3: 70% equilibration buffer 30% elution buffer

f) Elution: 50 mM sodium phosphate, 140 mM NaCl, 0.0005% t-80 (w:w), pH 6.4±0.1

Table 3: Buffer preparation per L for the polishing step: Toyopearl Ether-650M

| Buffer | Condition | Equilibration | Wash 1 | Elution |
|---|---|---|---|---|
| Volume | 1 L | 1 L | 1 L | 1 L |
| $Na_2HPO_4$ x 2 $H_2O$ 178 g/mol (g) | | | | 3.5 |
| $NaH_2PO_4$ x 2 $H_2O$ 156 g/mol (g) | | 7.8 | | 4.7 |
| NaCl (g) | | | | 8.2 |
| $NH_4Ac$ (77 g/mol) (g) | 254 | 123 | 254 | |
| $NH_4Cl$ (53.5 g/mol) | 139 | 64 | 123 | |
| Tween 80 (g) | 1 | 1 g of stock 0.5% | 5 | 1 g of stock 0.5% |
| $H_2O$ (g) | 671 | 849 | 683 | 988 |
| Glacial acetic acid (g) | 14.5 | | 5.3 | |
| pH | 6.1±0.1 | 6.4±0.1 | 6.5±0.1 | 6.4±0.1 |
| Conductivity (mS/cm) | 210±20 | 180±10 | 210±20 | 18±2 |

UFDF buffer:

Equilibration and diafiltration buffer: 3.7 mM sodium phosphate, 150 mM NaCl, 5 mM glycine, 10 mM mannitol, 0.0005% tween 80 (w:w), pH 6.2±0.15.

Table 4: Buffer preparation per L and concentration for the UFDF step.

| Buffer | Equilibration and diafiltration | Concentration |
|---|---|---|
| Volume | 1 L | |
| $Na_2HPO_4$ x 2 $H_2O$ 178 g/mol (g) | 0.161 | 0.9 mM |
| $NaH_2PO_4$ x 2 $H_2O$ 156 g/mol (g) | 0.437 | 2.8 mM |
| NaCl (g) | 8.762 | 150 mM |
| Tween 80 (g) | 1.0 of stock 0.5% | 0.0005 % |
| $H_2O$ (g) | 990 | |
| Glycin (g) | 0.375 | 5 |
| Mannitol (g) | 1.822 | 10 |
| pH | 6.2±0.1 | 6.2±0.1 |
| Osmolality (mOsm/kg) | 300 | 300 |

- Cleaning in place buffers:
- Cleaning buffer Capto Blue, Capto Adhere and UFDF: 1 M NaOH
- Cleaning buffer Toyopearl Ether: 0.5 M NaOH
- Neutralizing buffer all steps: 140 mM sodium phosphate, pH 6.6±0.2
- Storage buffer Blue, Adhere, Ether: 20% ethanol
- Storage buffer UFDF cassette: 0.1 M NaOH

Table 5: Buffer preparation per L of cleaning in place and storage buffers

| Buffer | 1 M NaOH | 0.5 M NaOH | 20% EtOH | 140 mM sodium phosphate |
|---|---|---|---|---|
| Volume | 1 L | 1 L | 1 L | 1 L |
| Sodium hydroxide (g) | 40 | 20 | | |
| 99% ethanol (g) | | | ~200 | |
| $Na_2HPO_4$ x 2 $H_2O$ 178 g/mol (g) | | | | 8.7 |
| $NaH_2PO_4$ x 2 $H_2O$ 156 g/mol (g) | | | | 14.2 |
| $H_2O$ (g) | to 1000 | to 1000 | ~800 | 1000 |
| pH | | | | 6.6±0.2 |

**In process analysis**

Enzymatic activity was measured by procedure 65; HNG assay for analyzing galactocerebrosidase (GALC). The in-house rhGALC standard StG01 is used for preapartion of a standard curve.

Protein concentration: Protein concentration was measured by procedure 75; Protein determination of rhGALC using Pierce 660 nm Protein Assay. Dilutions of the in-house standard StG02 as standard curve. Protein concentration of StG02 was determined externally by amino acid analysis (AAA) (Amino acid Analysis Center, Uppsala University, Sweden).

For information A280 was measured dividing the observed absorbance with the theoretical extinction coefficient 2.5 of human GALC.

Specific activity was calculated by dividing enzymatic activity with rhGALC protein concentration.

Identity was analyzed by procedure 70, Western Blot analysis of recombinant human galactocerebrosidase (rhGALC). A polyclonal antibody, purified on Protein A sepharose, generated against the in-house standard StG02 was used for detection.

Isoelectric focusing (IEF) was analyzed by procedure 74 on Novex IEF gel pH 3-10 to evaluate the isoelectric point of rhGALC. The final product was compared to the in-house rhGALC standard StG03 as an additional measure of identity.

Purity was analyzed by procedure 69 with SDS-PAGE (4-12% Bis-Tris NuPAGE, MOPS buffer) stained with Colloidal Blue.

Impurities were analyzed by procedure 43; ELISA method for determination of CHO host cell proteins.

Tween concentration was quantified by procedure 73 with a RP-HPLC method.

Native PAGE was performed as a measure of rhGALC formations for information. The analysis was performed according to instructions from the manufacturer (Invitrogen).

Osmolality (Vapro osmometer) and pH (Metrohm) were measured in the final product TG1106.

Carbohydrate composition was measured mainly by instruction 25 (other dilutions of the standard) by HPLC with fluorescence detection of 2-AA labeled released monosaccharides.

Harvest:

After closing the bioreactor, sodium acetate, pH 5 is added to the harvest in the bioreactor to keep pH <7, which is optimal for the enzyme. The pH stabilized harvest is pumped out from the bioreactor through depth filters buffer, to remove the cells. The filters are rinsed with conditioning buffer after the filtration. The final mix of harvest to conditioning buffer should be ~2:1 (w:w).

General information about the process conditions:

The equilibration, loading and equilibration wash of the chromatographic steps are performed with a peristaltic pump with maximum flow rate 100 mL/min, corresponding to 150 cm/hr. The remaining parts of the runs are performed with the Biological Duo-Flow system upgraded with Maximizer 80, with maximum flow rate 80 mL/min, corresponding to 125 cm/hr. The flow rates can be adjusted if a more appropriate chromatographic system is used. However, buffers with propylene glycol (PG) should be run with the indicated flow rate.

All chromatographic steps are run in binding mode and contain several wash steps. High concentration of PG is needed for elution of the hydrophobic rhGALC from the first two steps in the process. To remain enzymatic activity

the product needs to be collected into prefilled containers from these steps. No organic solvent is needed for elution from the polishing step.

All buffers, except Capto Blue conditioning buffer, contain detergent (tween 80), which appear necessary to keep the enzymatic activity of rhGALC. Harvest media contain pluronic, which replaces tween in the Capto Blue conditioning. Another motive for omitting tween in the conditioning is that it could cause opalescence of the harvest after ~>6 hours of storage at room temperature.

**Capto Blue**

Capto™ Blue is an affinity bioprocess media that binds through aromatic and electrostatic interactions.

The conditioned harvest is loaded onto the Capto Blue column within 24 hours from clarification. From a 20 L bioreactor three cycles of Capto Blue in pilot scale (740 mL) is needed. The column is washed with equilibration buffer and a wash buffer containing 10% propylene glycol (PG) and 5% isopropanol (IPA). Elution is performed with a buffer containing 50% PG into a prefilled container. Collection into a container filled with elution mix buffer has three purposes:

1) Decrease PG to remain enzymatic stability of rhGALC.
2) Act as a dedicated virus inactivation step. The final concentration of detergent was 1% and the product pool was stored for >16 hours at room temperature.
3) Condition the product pool for the next step in the DSP, Capto Adhere.

As described above the Blue product may be collected into an elution mix buffer. The final tween concentration is 1% and the pool is stored for 16-24 hours at room temperature as a dedicated virus inactivation step.

**Capto Adhere**

Capto™ Adhere is a strong anion exchange bioprocess media with multimodal functionalities. It binds through ionic interactions, hydrogen binding and hydrophobic interaction.

The product from Capto Blue is loaded in three sequential cycles after maximum 4 days hold time onto the Capto Adhere column. The hold time period starts with 18-20 hours at room temperature as a virus inactivation step. In case of longer hold time the product pool is moved to 5±3° C for the remaining time. It is moved back to room temperature 8-15 hours before the run to accumulate to room temperature. The product pool is loaded at pH 6.1. The pH is decreased fast to 4.7 by a wash at high ionic strength and IPA followed by a wash at low ionic strength. Elution is performed with buffer at pH 4.55 containing 40% PG into a prefilled container to increase pH and decrease PG concentration to keep the enzymatic activity of rhGALC.

**Toyopearl Ether**

Toyopearl Ether-650M is a methacrylic polymer (65 μm particle size) with high mechanical and chemical stability. Ether has the highest hydrophilicity in the Tosoh serie of hydrophobic interaction ligands and is designed for purification of very hydrophobic proteins.

The product from Capto Adhere is stored for maximum 24 hours at room temperature or 4 days at 5±3° C. If stored cold it is moved back to room temperature 8-15 hours before the run to accumulate to room temperature. It is mixed 1:2.5 (w:w) with the Ether conditioning buffer (three cycles) containing high ammonium acetate, ammonium chloride and tween concentrations. The Ether column is equilibrated with a buffer with 200x lower tween concentration compared to the conditioned start. After loading the column is washed with equilibration buffer. The next wash step increases tween and salt. It is followed by a long wash with the equilibration buffer to decrease the tween concentrations again and a wash with a mix of equilibration and elution buffer to decrease ammonium salts. Elution is performed with a sodium phosphate buffer containing low tween (0.0005%) and sodium chloride, at pH 6.4.

**Planova 15N Virus filtration**

In production scale the polishing step will be followed with nanofiltration through Planova 15N as a dedicated virus removal step.

**UFDF**

The three polishing product pools are pooled and formulated in one cycle of ultrafiltration/ diafiltration (UFDF) with tangential flow filtration (TFF) using a Pellicon polyether sulfone membrane with molecular weight cut off (MWCO)

30 kDa . The feed channels are type V with open channels and the cassette area is 0.1 $m^2$.
The pump is set to 230 rpm and transmembrane pressure (TMP) is 0.5 bar ($0.6_{in}/0.4_{out}$). The membrane is equilibrated with formulation buffer and the first volume of buffer is exchanged by dilution followed by concentration (UF) and 7 times diafiltration (DF). Totally eight volumes of buffer are exchanged.

**Filtration and filling**

The UFDF product (retentate) is filtered through a 0.22 μm PES filter under aseptic condition in a LAF bench. The final product is filled into sterile containers of various volumes (0.25-20 mL per vial/bottle). The final product is named TG1106 and is stored frozen at -80±10° C.

**Example 2**

Summary of results

The total yield for the downstream process from clarified harvest from the 20 L bioreactor to final product was 74% based on activity.
The total activity in ~19.5 L clarified harvest was 23 million Units. The total yield in the final product, TG1106, was 17 million Units or 1.0 g pure rhGALC.

**Activity yield and conditions**

The total yield (74%) and was calculated from clarified harvest to final product. The reason was that the clarification process was still not decided and not part of this study.
The chromatographic steps were run in three cycles. The three polishing step products were pooled and formulated in one UFDF. The UFDF product was sterile filtered and divided into containers. The yield, based on % activity for each step and cycle is shown in figure 2. Total activity from clarified harvest to final product is shown in figure 3.
The average yield for the capture step, Capto Blue, was 84±8.1%.
The average yield for the intermediate step, Capto Adhere, was 87±3.5%.
The average yield for the polishing step, Toyopearl Ether, was 76±0.6%*
The yield for the UFDF step was 117%.
The yield for the final sterile filtration was 102%.

**Analyses results**

In process samples and final product were analyzed by a set of analytical methods as described above. The table below summarizes the analytical results for final product TG1106.

| **Characterization** | **Analysis** | **Method** | **Result** |
|---|---|---|---|
| **Content** | Protein concentration | 660 nm | 2.5±0.3 mg/mL |
| | | *(A280 for information/2.5)* | *(2.7 mg/ml)* |
| | Enzymatic activity | HNG assay | 42.5±0.8 kU/mL |
| | Specific activity | HNG/660 nm | 16.9 kU/mg |
| **Identity** | Western blot | Detection with polyclonal antibody to rhGALC. | 80 kDa band approved |
| | IEF | pH 3-10 | pI=~6.35 approved |
| **Purity** | SDS-PAGE | Colloidal Blue staining | >99% |
| **Impurities** | Host cell proteins | HCP ELISA | 30 ng/mg rhGALC |

(continued)

| Characterization | Analysis | Method | Result |
|---|---|---|---|
| **Other** | Tween 80 | RP HPLC | 0.025% |
| | rhGALC formations | Native PAGE for information | Major formation: dimer ~7 forms visible from monomer to multimer |
| | pH | pH meter | 6.02 |
| | Osmolality | Vapro osmometer | 297 mOsm/kg |
| | Transmittance | T580 | 98.5% |
| | Carbohydrate | Monosaccharide composition for information | ~7% (w:w) 2-3 mol M6P/mol rhGALC<br>~12 mol MAN/mol rhGALC |

**Example 3**

Identity- Western blot

The purified product was identified as human GALC by western blot analysis.

The proteins in the polishing products, the UFDF product and final product were separated by SDS-PAGE and electrophoretically transferred to a polyvinylidene difluoride (PVDF) membrane. rhGALC was detected with a polyclonal rabbit anti GALC antibody generated against the in-house rhGALC standard StG02. The antibodies had been purified on a Protein A sepharose column (GE Healthcare). They detect 80 kDa GALC as well as the 50 kDa and the 30 kDa processed forms of GALC. A prestained protein ladder was used to verify the transfer and to estimate apparent molecular weight (MW).

Figure 4 shows a blot where the 80 kDa rhGALC was detected in the final product. No processed forms were detected at this protein load. The in-house standards, StG02 and StG03 were analyzed as references. The standards had been analyzed by amino acid analysis and their found amino acid compositions correlated with the theoretical composition of human GALC. Figure 5 was loaded with excess protein and in addition to 80 kDa rhGALC both the 50 and 30 kDa processed forms were identified as weak bands. An additional band, with apparent MW 160 kDa, was identified, probably rhGALC dimer that was not fully dissolved by SDS under reducing conditions.

**Example 4**

Isoelectric focusing

The isoelectric point of final product TG1106 was calculated to 6.35.

TG1106 and standard StG03 were separated according to charge on a pH 3-10 isoelectric (IEF) focusing gel. Electrophoresis was performed cold (on ice) at 100V for 1 hour, then 200V for 1 hour and finally 500V for 2 hours. The gel, shown in figure 6, was stained with Colloidal Blue. The calculated isoelectric point (pI) was estimated to 6.35, which was higher than the theoretical pI 5.9 of GALC. There was no difference between TG1106 and StG03.

**Example 5**

Purity-SDS-PAGE

The purity in final product TG1106 was estimated to >99%. Processed rhGALC was estimated to <0.5%.

The in process samples were separated, mainly according to size, by electrophoresis (SDS-PAGE), on a NuPAGE 4-12% Bis-Tris gel with MOPS buffer. rhGALC and potential impurities were visualized by Colloidal Blue. Colloidal Blue has a dynamic linear response that is independent on type of protein, meaning that as long as the protein concentrations are sufficient it is preferable for estimation of degree of purity compared to silver staining. The apparent molecular weight (MW) of rhGALC is 80 kDa, while processed forms have apparent molecular weights of 50 and 30 kDa.

Figure 7 shows a scan of in-process samples. As seen impurities were visualized after the Capto Blue and Capto Adhere steps, while only rhGALC was visualized after the Ether step. The band at ~160 kDa, may possibly be a dimer of rhGALC, since it is also identified by western blot (see also figure 6).

Figures 8 and 9 compare the final product TG1106 with the in-house standard StG03 for estimation of purity. No band for high load of TG1106, except rhGALC, had higher intensity than the lowest StG03. For the highest concentration of TG1106 a strange double band can be seen right below the rhGALC dimer, seen in both gels. This is most probably an artifact since it is not seen at all in the next highest load. Because of the strange double band at highest load the purity was calculated from the next highest load in figure 10; 100%-0.8% (0.14 μg/16 μg) =99.2% purity. At excessive load rhGALC processed forms 30 and 50 kDa could be visualized as low intensity bands. None of these bands had higher intensity than the 80 kDa band for the lowest standard meaning that processing was <0.5%.

The 160 kDa band might be an artifact. It could be that at high rhGALC concentration the SDS in the sample buffer is not sufficient for forming monomers of the rhGALC multimers (see Native PAGE, 7.2.5). But if not, estimation could be that the intensity of the "dimer" for TG1106 16 μg load is similar as the 80 kDa band for StG03 0.36 μg load, meaning ~2% of this form in the final product.

**Example 6**

Native PAGE

Native PAGE shows that the major formation of rhGALC is the dimer, but multimers with up to 10 rhGALC molecules exists.

Native PAGE was run for information of rhGALC formations at neutral pH. As seen in figure 10 rhGALC had a ladder of formations. Final product TG1106 was compared to rhGALC in-house standard StG03 and the pattern was similar. It could be that the lowest band (apparent MW 80-100 kDa) is rhGALC monomer and the second band is rhGALC dimer, followed by formations adding on more rhGALC monomers and/or dimers. Up to seven forms of rhGALC were visualized on the gel. rhGALC dimer was the most pronounced independent on load. An earlier electron microscopy study has verified that several forms exist of which the major form has a diameter of ~20 nm, which could correspond to rhGALC dimer.

**Example 7**

Impurities-HCP ELISA

Residual CHO cell host cell proteins (HCP) were quantified to 30 ng/mg rhGALC in final product TG1106.

Chinese hamster ovary (CHO) host cell proteins (HCP) were analyzed as a measure of impurities. Generic antibodies purchased from Cygnus Technologies were used in the ELISA. The antibodies were generated from cell proteins typically secreted (3G 0016-AF) as well as from intracellular proteins (C0016-PA) from CHO cells. The standard was prepared from 10% lysed and 90% secreted HCP from parental CHO (DG44 strain) cells.

HCP were measured after the Ether steps, the UFDF step and in the final product. The rhGALC in-house standard StG02 and Tox ASA were analyzed as references. As seen in table 23 the process reduced the HCP levels to 30 ng/mg rhGALC in final product TG1106. Residual HCP levels were similar in the Ether product and in the final product indicating that the UFDF step did not remove any additional HCP.

HCP levels after Ether, UFDF and in final product TG1106. StG02, StG03 and Tox ASA are references.

| Sample | Protein 660 nm | HCP | |
|---|---|---|---|
| | (mg/mL) | (ng/mL) | (ng/mg) |
| T03E | 0.47 | 15 | 32 |
| | | | |
| T04E | 0.48 | 9 | 19 |
| T05E | 0.56 | 14 | 25 |
| T01U | 2.66 | 98 | 37 |
| TG1106 | 2.52 | 79 | 30 |
| StG02 | 2.2 | 75 | 34 |
| StG03 | 1.42 | 172 | 121 |
| Reference | 6.1 | 68 | 11 |

**Example 8**

Monosaccharide composition

The preliminary degree of glyscosylation was estimated to 7% and each mol rhGALC contained 2-3 mol mannose-6-phoshate residues.

Only one preliminary analysis was performed of final product TG1106. It indicates that rhGALC has ~7 % carbohydrates (w:w). The glycosylation is most likely of high mannose type.

The preliminary data indicate the following mol of each monosaccharide per mol rhGALC:

| | |
|---|---|
| Glucose amine (GLCN): | 9 mol/mol |
| Galactoseamine (GALN) | 0-0.3 mol/mol |
| Galactose (GAL) | 1 mol/mol |
| Mannose (MAN) | 12 mol/mol |
| Mannose-6 phosphate (M6P) | 2-3 mol/mol |
| Fucose (FUC) | 0-0.5 mol/mol |

**Discussion**

The optimized three chromatographic step process described in here produced a pure rhGALC product that fulfills quality requirements for animal studies and most likely also clinical studies. DNA remains to be analyzed.

The yield from bioreactor B5:19, based on enzymatic activity from clarified harvest to final product was 74%. Having in mind that GALC is a very hydrophobic protein this was especially satisfactory.

The clarification of harvest was the only step without acceptable yield. Since there are possible improvements to be evaluated the DSP yield was calculated from clarified and conditioned harvest. Around 20% activity was lost by the depth filtration. An earlier study indicated that TFF could improve the clarification yield. The total yield, including the clarification, was 58%.

The theoretical pI of GALC is 5.9, while the found pI was 6.3. Since rhGALC contains acid M6P and sialic acid this was surprising. Experiments, prior to this study, have shown rhGALC to be very pH sensitive; for long storage periods it is only stable around its pI; pH 6.0-6.6. Theoretically, pH around the pI should be avoided in a DSP to avoid precipitation, but for the rhGALC DSP it is the only possibility. The product has been clear throughout the DSP and no tendency of opalescence has been seen. An explanation for the high pI could be that tween/rhGALC micelle formations alter the exposed amino acids from the predicted. Tween was included in all DSP buffers (except Blue conditioning, where it is replaced by pluronic) to maintain enzymatic activity. The minimum tween concentration for keeping the stability remains to be evaluated.

Elution from both the capture and intermediate steps were complicated. Propylene glycol (PG) was a prerequisite for both elution buffers. In addition, other additives and careful optimization of the buffers were required for optimal yield. A drawback could be that highly hydrophobic contaminants may co-elute with rhGALC. Prosaposin, a 70 kDa hydrophobic protein, was identified (western blot with a saposin A antibody) as contaminant after the capture and the intermediate step.

High concentration of PG is not optimal for rhGALC enzymatic activity and thus collection was in prefilled containers.

Some additional stepwise comments to the process:

The capture step, Capto™ Blue, stabilized the enzyme and removed the media color, which was its major goal. Of major importance for the yield was to prepare the elution buffer correctly. It must contain 50% (v:v) propylene glycol, which corresponds to 521 g/L buffer.

The intermediate step, Capto™ Adhere, removed the bulk of contaminants. Acidic conditions were needed for elution. To avoid precipitation of protein it was of importance to change to acidic conditions fast, by a buffer with high ionic strength. The buffer was then changed to an acidic buffer with low ionic strength. The reasons were that additional impurities were removed and to assure that the eluted product was acidic, but with low ionic strength to be able to fast change pH back >6 by collection into a prefilled container with pH 6.5 buffer.

Toyopearl Ether combined a sufficient yield using aqueous buffers, without any organic solvents such as PG and IPA, with the possibility to remove the final HCP with hydrophobic characteristics. An advantage was that elution with acceptable yield was possible with a phosphate buffer with sodium chloride. A drawback was that extreme salt concentrations were needed for binding of rhGALC. A large volume of conditioning buffer was needed, making the loading time long.

This step was powerful in separating rhGALC from contaminants with similar characteristics as rhGALC. Of importance for a robust clearance of contaminants was the repeated alteration, both in tween and salt concentrations, which could be described as wash and rinse cycles. No prosaposine could be identified after the Ether step.

Two dedicated virus inactivation/removal steps are part of the process in productions scale. No virus spiking experiments have been performed, but regarding yield and flux the steps look promising.

A detergent inactivation step was combined with the elution from the capture step. The high tween concentration had no negative influence on the binding to the intermediate column and the enzymatic activity remained after storage at room temperature for 24 hours (also if combined with 3 days storage at +5° C). The plan is to have a virus filtration step, Planova 15N, after the Ether step. This was found feasible in an earlier study and it was not repeated. Preliminary large scale estimation approximates that 80 L product can be filtered through 1 $m^2$ Planova 15N in ~5 hours.

UFDF, with a V-screen 30 kDa MWCO filter, was used for formulation and concentration after the polishing step. The V screen filter, with open channels, is only available in pilot scale (0.1 $m^2$) that requires large amounts of product for optimization studies. The conditions have been modified in small steps based on the three UFDF runs in the previous study. A drawback with UFDF could be that tween accumulates. The Ether products were washed and eluted with buffer containing only 0.0005% tween. It was difficult to quantify the tween in the Ether product, but an estimate was ~0.003%. The UFDF/formulation buffer contained 0.0005% tween. After 8 volumes of buffer exchange and ~7 times concentration the tween concentration was ~0.025%. The UFDF product was easily 0.22 ~μm filtered with no loss of product into the final product.

Preliminary data indicate that this tween concentration in combination with the other components of the formulation buffer is optimal for keeping rhGALC stability for long term storage at +5, -20, -80 °C. It is possible to freeze dry the product, if mannitol is increased to 250 mM.

SDS-PAGE followed by Colloidal Blue staining showed a pure final product; >99%. The processed 30 and 50 kDa forms of rhGALC could be seen as minor bands and were estimated to <0.5%. The most pronounced (~2%) visualized protein apart from the 80 kDa rhGALC form was a 160 kDa rhGALC form seen only if high concentration of rhGALC was mixed with SDS-PAGE sample buffer. Western blot analysis verified that the protein contained rhGALC, maybe as dimer that was not sufficiently dissolved by SDS under reduced conditions. Maybe this "dimer" was only an artifact. Native PAGE, at neutral pH, indicated that rhGALC has several formations, from monomers to formations of rhGALC multimers of ~10 molecuels. The most common formation seemed to be the dimer

## Conclusion and summary

Recombinant human GALC, expressed in CHO cells, were cultured in one 20 L bioreactors at the Royal Institute of Technology, Stockholm, Sweden.

19.5 L harvest was purified to 17 million Units or 1.0 g pure rhGALC with a downstream process consisting of three chromatographic steps; Capto Blue, Capto Adhere and Toyopearl Ether, all run in binding mode. A virus inactivation step consisting of 16-24 hours incubation at room temperature with 1% tween 80 was performed after Capto Blue. The product was formulated by tangential flow filtration and sterile filtered to the final product TG1106.

The harvest was stabilized by addition of sodium acetate to the bioreactor before clarification by depth filtration and conditioning for binding to the capture column, Capto Blue. The conditioned harvest was loaded onto the 730 mL Capto Blue column in three cycles performed within 24 hours. The product was eluted with a 50% propylene glycol buffer into a "three-purpose" buffer to reduce propylene glycol, increase tween as a dedicated virus inactivation step and condition the start for the next step. The conditioned start was loaded onto the 730 mL Capto Adhere column in three sequential cycles. The product was eluted with acidic pH and propylene glycol into a buffer that reduced propylene glycol and increased pH to keep the activity. After additional mixing with a buffer with ammonium acetate and ammonium chloride the conditioned start was loaded onto the 540 mL Toyopearl Ether column in three sequential runs. The product was eluted in a phosphate buffer containing sodium chloride and a low tween concentration. The plan is to include a virus filtration step after the polishing step, but it was excluded in this study. The polishing products were pooled, buffer changed and concentrated by UFDF to a formulation buffer optimal for long term storage of pure rhGALC. The UFDF product was sterile filtered into the final product.

The final product was analyzed by a set of analytical methods. The protein concentration is 2.5 mg/mL and the enzymatic activity 42.5 kU/mL, resulting in a specific activity of 16.9 kU/mg. The estimated purity is >99%. Residual HCP are 30 ng/mg rhGALC. The final product is clear and colorless.

In conclusion, the new optimized DSP has been scaled up successfully to pilot scale resulting in a yield of 74%, based on activity. The final product TG1106 fulfills quality requirements for animal studies.

**Example 9**

Examples with ionic separation:

Ionic separation was tested as described below with satisfactory results.

1) Mustang Q (MQ) is a disposable membrane with anionic support. It was tested in combination with the current process in flow through mode (impurities such as DNA and host cell proteins bind to the membrane and GALC flows through). It was tested either before the Ether step or after the UFDF (formulation step. It was also tested in line with Capto Blue, also in flow through mode.

a) when included after the Ether step: Equilibration of MQ was performed with 3.7mM sodium phosphate, 5mM glycine, 10mM mannitol, 0.075 M NaCl 0.0005% tween 80, pH 6.2 Product was diluted 1:1 (v:v) with 3.7mM sodium phosphate, 5mM glycine, 10mM mannitol, 0.0005% tween 80, pH 6.2 (or the same buffer with 0.15 M NaCl) before running it through MQ.
b) When included after the UFDF step. MQ was equilibrated with 3.7mM sodium phosphate, 0.2 M NaCl, 5mM glycine, 10mM mannitol, 0.0005% tween 80, pH 6.2 Product was diluted with 1M NaCl until conductivity was 20 mS/cm (approximately 1 volume product: 0.2 volume 1 M NaCl) before the product was run through MQ. Before sterile filtration the product was diluted with formulation buffer without NaCl to bring conductivity back to 15 mS/cm (0.15 M NaCl).

Outline of process:

Clarified harvest
Capto Blue
Virus inactivation
Capto Adhere
Toyopearl Ether
Alternative a) Mustang Q
UFDF
Alternative b) Mustang Q
0,22 μm filter

2) A strong anion exchange (AIEX) resin, such as Capto Q, Giga Cap Q, Q FF, can be included in the downstream process in binding mode. It is also possible to include a weak anion exchange resin such as Capto DEAE and DEAE FF but the remaining impurities were found higher. It may be used before or after the polishing step (hydrophobic interaction (HIC)).

Procedure 1:
Outline of process:

Clarified harvest
Capto Blue
Virus inactivation
Virus inactivation 15% IPA and 1% t80. Note: some inactivation when IPA is used.
Capto Adhere
Macroprep Methyl
Equilibrated (Eq) with 0.4-0.6 M $(NH_4)_2SO_4$, 5% glycerol, 0.1% tween 80 (t80), pH 6.5. Adhered product conditioned with 20 mM NaPi , 5% glycerol, 0.8-1.2 M $(NH_4)_2SO_4$ pH 6.5 (1:1) and load.

Wash 1: 0.6 M NaPi, 0.1% t80, pH 6.5.
Wash 2: 20 mM NaPi, 15 mM NaCl, 0.1% T80,

Giga Cap Q
Equilibrated with 40 mM MES, 15 mM NaCl, 5% glycerol, 0.1% T80, pH 6.5. Loaded Methyl product (no conditioning) and washed with eq buffer. Elution: 40 mM MES, 0.7 M NaCl, 5% glycerol
UFDF

Procedure 2:
Outline of process:

Clarified harvest
Capto Blue
Virus inactivation
Capto Adhere
Giga Q
Equilibrated with 40 mM MES, 15 mM NaCl, 0.1% T80, pH 6.5. Capto adhere conditioned with 20 mM NaPi, 0.1% t80 pH 6.5 to conductivity ··7 mS/cm. Loaded and washed with eq buffer.
Ether
UFDF

**Example 10**

The following multimodal resins were tested with satisfactory results:

1) MMC Capture (very early experiments)
Start: pH of harvest was adjusted to 5.6 - 6.0 w. 400 mM Na-Pi (acidic). Equilibration buffer used: 20 mM Na-Pi pH 5.6 - 6.0 + 0.1 M NaCl + 0.05% Tween 80 (t80)
Wash buffer used: 0.95 M Na-Ac pH 4.9 + 5% IPA
Elution buffer used: 50 mM Tris-HCl pH 9.0 + 1.0M NaCl + 40 % Prop Glycol + 0.1% t80.
Results: Presence of enzyme was analysed using Dot-Blot method. Enzyme was detected in Start (+++) and in Elution - fraction 1 (++). No enzyme detected in flow through. SDS-PAGE and HPLC analyses were performed on fractions
2) Butyl-S as 2$^{nd}$ intermediate step or as polishing step:

Start: Condition to 0.5-6 M $(NH4)_2SO_4$.
Equilibration: 20 mM NaPi 0.5 M$(NH_4)_2SO_4$.5% glycerol, 0.1% t80 pH 6.5
Wash: Equlibration buffer
Elution: 20 mM NaPi, 0.1 M NaAc, 5% IPA, 0.1% t80, pH 7.8

3) PPG as 2$^{nd}$ intermediate step (ex test 44-47)
Start: Condition to 0.5 M $(NH_4)_2SO_4$ and 0.5 M NaAc, pH 6.3
Eq: 20 mM NaPi 0.5 M $(NH_4)_2SO_4$ and 0.5 M NaAc, 0.0005-0.1% t80, 5%glycerol, pH 6.4
Wash: 1.6 M NaAc, 0.1% t80, pH 7.4
Wash 2: 0.7 M naPi, pH 6.5
Elution: 20 mM NaPi, 30% prolyleneglycol, 0.05% t80, pH 7.8

**Example 11**

The following combinations of resins were tested with satisfactory results:

Preliminary test: average approximately 350 ng HCP/mg GALC
Capto Blue
Capto adhere
Capto Butyl
Capto DEAE

Test 1: 240 ng HCP/mg GALC
Capto Blue
Capto adhere
Butyl-S

Test 2: 430 ng HCP/mg GALC
Capto Blue
Capto Adhere
Macro-Prep Methyl

Giga Cap Q

Test 3: 78 ng HCP/mg GALC
Capto Blue
Capto adhere
PPG

Test 4: 50 ng HCP/mg GALC (but opalescence due to virus inactivation with IPA+tween)
Capto Blue
Capto Adhere
Butyl-S

Test 5: 193 ngHCP/mg GALC
Capto Blue-Mustang Q in line filter
Capto adhere
Ether

Test 6: 79 ngHCP/mg GALC
Capto Blue-Mustang Q in line
Capto adhere
Ether

**Example 12**

HNG assay for analyzing galactocerebrosidase (GALC) activity

Principle

Galactocerebrosidase (GALC) is responsible for the lysosomal catabolism of galactocerebroside (=galactosylceramide), a major lipid in myelin, kidney and epithelial cells. GALC hydrolyzes the galactose ester bonds of galactocerebroside, galactosylsphingosine, lactosylceramide, and monogalactosyldiglyceride. GALC is also able to hydrolyze the synthetic analogue of galactocerebroside, a chromogenic substrate, 2-hexadecanoylamino-4-nitrophenyl-b-D-galactopyranoside (HNG). The sodium salt of the product of the reaction, 2-hexadecanoylamino-4-nitrophenol (HN), absorbs light at 410 nm. This principle is utilized in the method described herein.

Analysis principle for GALC. HNG is hydrolyzed by GALC into HN (at pH 4.5), a yellow colored product (at pH 10.5), which is determined spectrophotometrically at 410 nm.

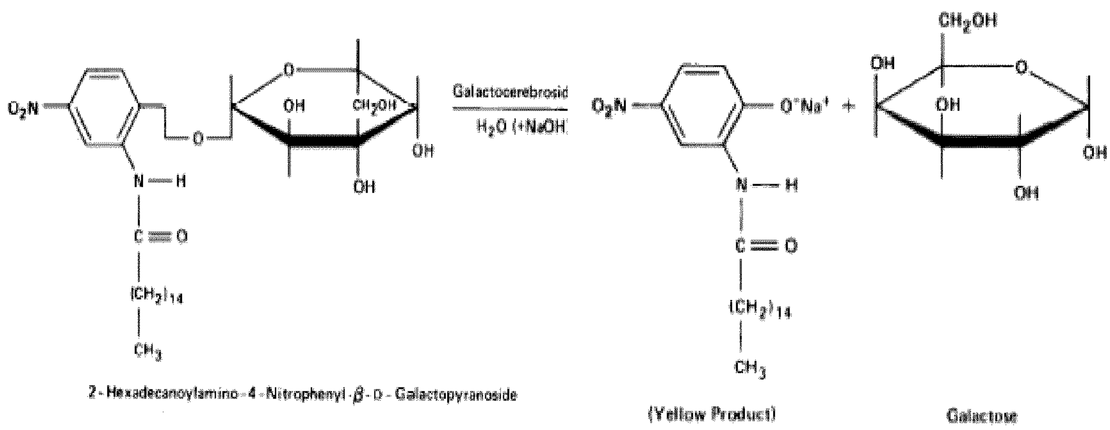


2-Hexadecanoylamino-4-Nitrophenyl-β-D-Galactopyranoside

(Yellow Product)

Galactose

Hydrolytic cleavage of 2-hexadecanoylamino-4-nitrophenyl-b-D-galactopyranoside Sample preparation

<u>Harvest or purified GALC</u>

Desired dilutions of samples were prepared using 0.5% Triton X-100. At least a dilution 1:10 was required for analysis.

Cell-lysate

Cell-pellets were washed in PBS, pelleted by centrifugation (400 x g for 5 min at RT) and lysed in 0.5% Triton X-100. Protein determination of cell-lysate was performed using the BCA Protein Assay Kit Microtiter Plate Protocol (Pierce).
A typical experiment was performed using 1-5mg cell-lysate proteins.

Preparation of a standard curve and assay control

The first in-house rhGALC standard StG01 was used for preparation of a standard curve. True replicas of five dilutions (1/400, 1/600, 1/800, 1/1200 and 1/1600) were prepared.

| Step no | Sample used for dilution | Sample (μl) | Pipette No* | 0.5% Triton X-100 (μl) | Pre-dilutions |
|---|---|---|---|---|---|
| 1 | StG01 | 10 | 1 | 90 | 1/10 |
| 2 | Pre-dilution 1/10 | 20 | 2 | 180 | 1/100 |
| | | | | | **Final dilutions** |
| 3 | Pre-dilution 1/100 | 50 | 2 | 150 | 1/400 |
| 3 | Pre-dilution 1/100 | 30 | 2 | 150 | 1/600 |
| 3 | Pre-dilution 1/100 | 25 | 2 | 175 | 1/800 |
| 3 | Pre-dilution 1/100 | 10 | 1 | 110 | 1/1200 |
| 3 | Pre-dilution 1/100 | 10 | 1 | 150 | 1/1600 |

The second rhGALC standard StG02 was used for preparation of an assay control:

| Step no | Sample used for dilution | Sample (μl) | Pipette No* | 0.5% Triton X-100 (μl) | Pre-dilutions |
|---|---|---|---|---|---|
| 1 | StG02 | 10 | 1 | 90 | 1/10 |
| 2 | Pre-dilution 1/10 | 10 | 1 | 190 | 1/200 |
| 3 | Pre-dilution 1/200 | 10 | 1 | 190 | 1/4000 |
| | | | | | **Final dilution** |
| 4 | Pre-dilution 1/4000 | 50 | 2 | 150 | 1/16000 |

Incubation procedure

a. 20 μl of standard dilutions, sample, control and blank (0.5% Triton X-100) were added to a U-type 96-well plate. 20 μl of each replica of the StG01 standard dilutions, control and samples (20 μl x 2 if a single dilution is prepared) and 20 μl x 2 of blank were used. When all wells were loaded 20μl HNG-assay substrate was added to all wells containing sample, blank and control followed by mixing. The plate was incubated at 37°C for 30 min or 1h.

b. 80 μl HNG stop solution (0.1 M glycine/0.1 M NaOH pH 10.5) was added using a multipipette followed by mixing on a plate-shaker for approximately 30 sec. and addition of160 μl ethanol. The solution was mixed and 200 μl was withdrawn from each well and transfered to wells of a 96-well filter plate sitting on a flat-bottomed 96-well plate. The plate was centrifuged at 2000 x g for 2 min at room temperature.

Measurements of A410nm

Measurements were performed using Spectra Max Plus plate reader or a BioTek plate reader.

Calculations

Definition: One unit (1 U) of enzyme activity was defined as the hydrolysis of 1 nmol HN per minute at 37°C, pH 4.5.

The mean HNG-activity of the first in-house rhGALC standard StG01 was set to 1884 U/ml. The activities used for the standard curve were calculated from dilutions of the mean StG01 HNG-activity:

| Standard no | Dilution of StG01 | Activity of diluted StG01 U/ml |
|---|---|---|
| 1 | 1/400 | 4.71 |
| 2 | 1/600 | 3.14 |
| 3 | 1/800 | 2.355 |
| 4 | 1/1200 | 1.57 |
| 5 | 1/1600 | 1.1775 |

Calculation of specific activity

The concentration of GALC in mixed samples was determined using GALC ELISA. Protein concentration in purified preparations of GALC was determined using A280 (the theroretical specific absorption coefficient for rhGALC is 2.5) or the 660 nm Protein Assay.

To calculate the specific activity (Units/mg) or the enzymatic activity per mg protein (Units/mg protein), the HNG activity was divided with the concentration of GALC or protein.

Originally, the GALC activity of cell-lysate has been defined as nmol hydrolyzed product per mg per hour (nmol/mg/h). To convert Units/mg to nmol/mg/h multiply by 60 (i.e. convert minutes to hour).

## Claims

1. A process for purifying recombinant human Galactocerebroside β-Galactosidase (rhGALC) from a cell culture, wherein a fraction of said cell culture comprising rhGALC is subjected to chromatography on resins, the process comprising

   a) A capture step in which said rhGALC is purified on a first multimodal chromatographic resin which binds through at least hydrophobic and electrostatic interactions and which comprises electrostatic ligands, optionally followed by virus inactivation and/or purification by ionic separation;
   b) An intermediate step in which said rhGALC is purified on a second multimodal chromatographic resin comprising an anionic and hydrophobic ligand, optionally followed by virus inactivation and/or purification by ionic separation;
   c) A polishing step in which said rhGALC is purified on a third chromatographic resin which is selected from the group consisting of a multimodal chromatography resin, an anion exchange resin and a hydrophobic interaction chromatography (HIC) resin and wherein the third chromatographic resin comprises a ligand comprising an ether group.

2. The process according to claim 1, wherein said intermediate step comprises purification of said rhGALC on a said second multimodal chromatographic resin, followed by purification of said rhGALC on a chromatography resin selected from the group consisting of:

   i) a multimodal chromatography resin which is different from said first and said second multimodal chromatographic resins; and
   ii) a hydrophobic interaction chromatography (HIC) resin.

3. The process according to claim 2, wherein said first and second multimodal chromatography resins are different resins.

4. The process according to any of the preceding claims, wherein said rhGALC is eluted from said first multimodal chromatographic resin in first elution buffer comprising at least 30% propylene glycol and/or ethylene glycol (v/v).

5. The process according to any of the preceding claims, wherein said rhGALC is eluted from said second multimodal chromatographic resin in a second elution buffer comprising at least 30% propylene glycol and/or ethylene glycol (v/v)) and having a pH below 5.5.

6. The process according to any of the preceding claims, said process comprising

   a) providing a fraction of said cell culture comprising rhGALC;
   b) loading the fraction of said cell culture onto a first multimodal chromatographic resin which binds through at least hydrophobic and electrostatic interactions and which comprises electrostatic ligands;
   c) eluting rhGALC from the first multimodal chromatographic resin in a first elution buffer comprising at least 30% propylene glycol and/or ethylene glycol (v/v) thereby providing a first eluate;
   d) loading the first eluate onto a second multimodal chromatographic resin comprising an anionic and hydrophobic ligand;
   e) eluting rhGALC from the second multimodal chromatographic resin in a second elution buffer comprising at least 30% propylene glycol and/or ethylene glycol (v/v) and having a pH below 5.5, thereby providing a second eluate;
   f) loading the second eluate onto a third chromatographic resin having hydrophobic ligands; and
   g) eluting rhGALC from the third chromatographic resin comprising a ligand comprising an ether group, which is selected from the group consisting of a multimodal chromatography resin, an anion exchange resin and a hydrophobic interaction chromatography (HIC) resin in an aqueous buffer, thereby providing a third eluate.

7. The process according to any of the preceding claims, wherein the first multimodal chromatographic resin comprises as ligand a compound of the formula (I), (II) or (III):

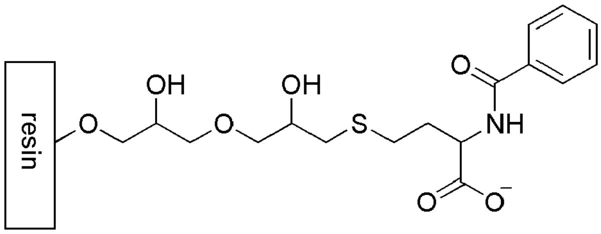

(I)

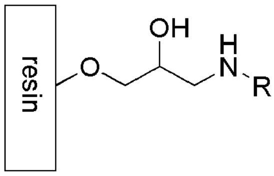

(II)

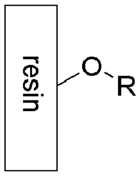

(III)

wherein R of the substances of formula (II) and (III) is a functional group of formula (IV):

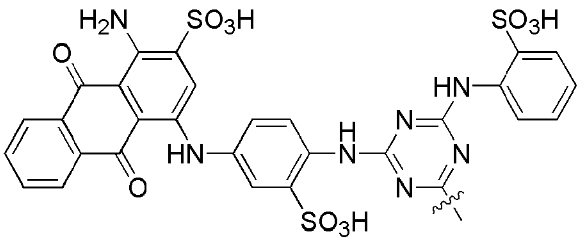

(IV).

8. The process according to any of the preceding claims, wherein the first chromatographic resin is washed in a wash buffer comprising at the most 20% of propylene glycol and/or ethylene glycol (v/v).

9. The process according to any of the preceding claims, wherein the first elution buffer comprises a total concentration of propylene glycol and/or ethylene glycol (v/v) of 40-60%.

10. The process according to any of the preceding claims, wherein the second multimodal chromatographic resin comprises a ligand of the formula (VIII):

[RESIN]- 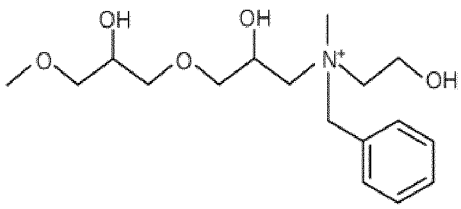

(VIII)

or a ligand of the formula (IX):

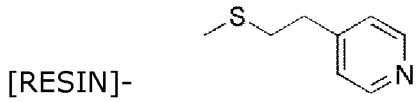

(IX)

**11.** A composition comprising rhGALC, wherein the molar ratio between full length rhGALC (80 kDa) and the main processed products (50 + 30 kDa) in said composition is at least 50:2.5.

**12.** The composition according to claim 11 which is obtainable by the purification process according to any of the preceding claims.

**13.** The composition according to any of claims 11-12 for use as a medicament.

**14.** The composition according to any of claims 11-12 for use in the treatment of Globoid Cell Leukodystrophy (Krabbe disease).

**Patentansprüche**

**1.** Verfahren zum Aufreinigen einer rekombinanten humanen Galactocerebrosid-β-galactosidase (rhGALC) aus einer Zellkultur, wobei eine Fraktion der Zellkultur, die rhGALC umfasst, einer Chromatographie an Harzen ausgesetzt ist, wobei der Verfahren Folgendes umfasst:

a) Einen Abfangschritt, bei dem die rhGALC auf einem ersten multimodalen Chromatographieharz aufgereinigt wird, das durch zumindest hydrophobe und elektrostatische Interaktionen bindet und das elektrostatische Liganden umfasst, gegebenenfalls gefolgt von einer Virusinaktivierung und/oder einer Aufreinigung durch eine ionische Trennung;
b) Einen Zwischenschritt, bei dem die rhGALC auf einem zweiten multimodalen Chromatographieharz aufgereinigt wird, das einen anionischen und hydrophobe Liganden umfasst, gegebenenfalls gefolgt von einer Virusinaktivierung und/oder einer Aufreinigung durch eine ionische Trennung;
c) Einen Polierschritt, bei dem die rhGALC auf einem dritten Chromatographieharz aufgereinigt wird, das aus der Gruppe bestehend aus einem multimodalen Chromatographieharz, einem Anionenaustauscherharz und einem Hydrophobinteraktionschromatographieharz (hydrophobic interaction chromatography resin - HIC-Harz) ausgewählt ist, und wobei das dritte Chromatographieharz eine Ethergruppe umfasst.

**2.** Verfahren nach Anspruch 1, wobei der Zwischenschritt eine Aufreinigung der rhGALC an einem zweiten multimodalen Chromatographieharz gefolgt von einer Aufreinigung der rhGALC an einem Chromatographieharz umfasst, das aus der Gruppe ausgewählt ist, die aus Folgendem besteht:

i) einem multimodalen Chromatographieharz, das sich von dem ersten und zweiten multimodalen Chromatographieharz unterscheidet; und
ii) einem Hydrophobinteraktionschromatographieharz (hydrophobic interaction chromatography resin - HIC-Harz).

**3.** Verfahren nach Anspruch 2, wobei es sich bei dem ersten und zweiten multimodalen Chromatographieharz um verschiedene Harze handelt.

**4.** Verfahren nach einem der vorangehenden Ansprüche, wobei die rhGALC von dem ersten multimodalen Chromatographieharz in einem ersten Elutionspuffer eluiert wird, der zumindest 30 Vol-% Propylenglykol und/oder Ethylenglykol umfasst.

**5.** Verfahren nach einem der vorangehenden Ansprüche, wobei die rhGALC von dem zweiten multimodalen Chromatographieharz in einem zweiten Elutionspuffer eluiert wird, der zumindest 30 Vol-% Propylenglykol und/oder Ethylenglykol umfasst und einen pH-Wert von unter 5,5 aufweist.

**6.** Verfahren nach einem der vorangehenden Ansprüche, wobei der Verfahren Folgendes umfasst:

a) Bereitstellen einer Fraktion der Zellkultur, die rhGALC umfasst;
b) Laden der Fraktion der Zellkultur auf ein erstes multimodales Chromatographieharz, das durch zumindest hydrophobe und elektrostatische Interaktionen bindet und das elektrostatische Liganden umfasst;
c) Eluieren der rhGALC von dem ersten multimodalen Chromatographieharz in einem ersten Elutionspuffer, der zumindest 30 Vol-% Propylenglykol und/oder Ethylenglykol umfasst, wodurch ein erstes Eluat bereitgestellt wird;
d) Laden des ersten Eluats auf ein zweites multimodales Chromatographieharz, das einen anionischen und hydrophoben Liganden umfasst;
e) Eluieren der rhGALC von dem zweiten multimodalen Chromatographieharz in einem zweiten Elutionspuffer, der zumindest 30 Vol-% Propylenglykol und/oder Ethylenglykol umfasst und einen pH-Wert unter 5,5 aufweist, wodurch ein zweites Eluat bereitgestellt wird;
f) Laden des zweiten Eluats auf ein drittes multimodales Chromatographieharz, das hydrophobe Liganden aufweist; und
g) Eluieren der rhGALC von dem dritten Chromatographieharz, das einen Liganden umfasst, der eine Ethergruppe umfasst, die aus der Gruppe bestehend aus einem multimodalem Chromatographieharz, einem Anionenaustauscherharz und einem Hydrophobinteraktionschromatographieharz (hydrophobic interaction chromatography resin - HIC-Harz) in einem wässrigen Puffer ausgewählt ist, wodurch ein drittes Eluat bereitgestellt wird.

**7.** Verfahren nach einem der vorangehenden Ansprüche, wobei das erste multimodale Chromatographieharz einen Liganden einer Verbindung der Formel (I), (II) oder (III) umfasst:

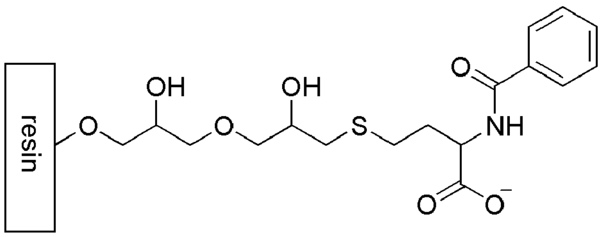

(I)

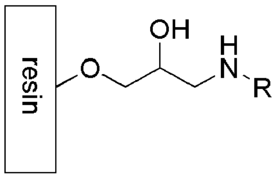

(II)

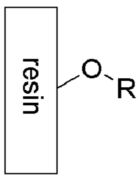

(III)

wobei R der Substanzen der Formel (II) und (III) eine funktionale Gruppe der Formel (IV) ist:

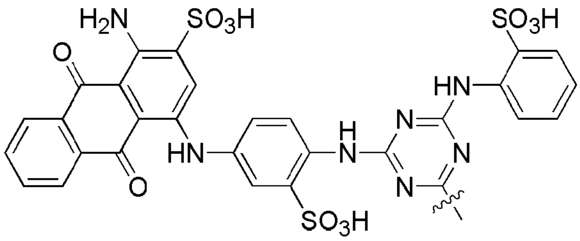

(IV).

**8.** Verfahren nach einem der vorangehenden Ansprüche, wobei das erste Chromatographieharz in einem Waschpuffer

gewaschen wird, der höchstens 20 Vol-% Propylenglykol und/oder Ethylenglykol umfasst.

**9.** Verfahren nach einem der vorangehenden Ansprüche, wobei der erste Elutionspuffer eine Gesamtkonzentration von Propylenglykol und/oder Ethylenglykol von 40-60 Vol.-% umfasst.

**10.** Verfahren nach einem der vorangehenden Ansprüche, wobei das zweite multimodale Chromatographieharz einen Liganden der Formel (VIII):

[HARZ]- 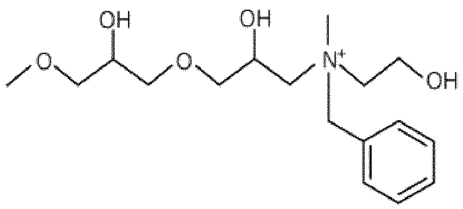 (VIII)

oder einen Liganden der Formel (IX):

[HARZ]- 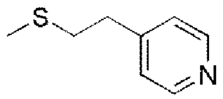 (IX)

umfasst.

**11.** Zusammensetzung, umfassend rhGALC, wobei das Molverhältnis der rhGALC voller Länge (80 kDa) zu dem wichtigsten verarbeiteten Produkten (50 + 30 kDa) in der Zusammensetzung mindestens 50:2,5 ist.

**12.** Zusammensetzung nach Anspruch 11, die durch den AufreinigungsVerfahren nach einem der vorangehenden Ansprüche erhalten werden kann.

**13.** Zusammensetzung nach einem der Ansprüche 11-12 zur Anwendung als ein Arzneimittel.

**14.** Zusammensetzung nach einem der Ansprüche 11-12 zur Anwendung bei der Behandlung von Globoidzellen-Leukodystrophie (Krabbe-Syndrom).

**Revendications**

**1.** Procédé pour purifier une Galactocérébroside β-Galactosidase recombinante humaine (rhGALC) à partir d'une culture cellulaire, dans lequel une fraction de ladite culture cellulaire comprenant de la rhGALC est soumise à la chromatographie sur résines, le procédé comprenant

a) une étape de capture dans laquelle ladite rhGALC est purifiée sur une première résine chromatographique multimodale qui se lie par au moins des interactions hydrophobes et électrostatiques et qui comprend des ligands électrostatiques, facultativement suivie par une inactivation virale et/ou une purification par séparation ionique ;
b) une étape intermédiaire dans laquelle ladite rhGALC est purifiée sur une deuxième résine chromatographique multimodale comprenant un ligand anionique et hydrophobe, facultativement suivie par une inactivation virale et/ou une purification par séparation ionique ;
c) une étape de polissage dans laquelle ladite rhGALC est purifiée sur une troisième résine chromatographique qui est sélectionnée parmi le groupe constitué par une résine de chromatographie multimodale, une résine d'échange d'anions et une résine de chromatographie d'interaction hydrophobe (HIC) et dans lequel la troisième résine chromatographique comprend un ligand comprenant un groupe éther.

**2.** Procédé selon la revendication 1, dans lequel ladite étape intermédiaire comprend la purification de ladite rhGALC sur une dite deuxième résine chromatographique multimodale, suivie par la purification de ladite rhGALC sur une résine de chromatographie sélectionnée parmi le groupe constitué par :

i) une résine de chromatographie multimodale qui est différente de ladite première et de ladite deuxième résines chromatographiques multimodales ; et
ii) une résine de chromatographie d'interaction hydrophobe (HIC).

**3.** Procédé selon la revendication 2, dans lequel lesdites première et deuxième résines de chromatographie multimodales sont des résines différentes.

**4.** Procédé selon l'une quelconque des revendications précédentes, dans lequel ladite rhGALC est éluée de ladite première résine chromatographique multimodale dans un premier tampon d'élution comprenant au moins 30 % de propylène glycol et/ou d'éthylène glycol (v/v).

**5.** Procédé selon l'une quelconque des revendications précédentes, dans lequel ladite rhGALC est éluée de ladite deuxième résine chromatographique multimodale dans un deuxième tampon d'élution comprenant au moins 30 % de propylène glycol et/ou d'éthylène glycol (v/v)) et ayant un pH inférieur à 5,5.

**6.** Procédé selon l'une quelconque des revendications précédentes, ledit procédé comprenant

a) la fourniture d'une fraction de ladite culture cellulaire contenant de la rhGALC ;
b) le chargement de la fraction de ladite culture cellulaire sur une première résine chromatographique multimodale qui se lie par au moins des interactions hydrophobes et électrostatiques et qui comprend des ligands électrostatiques ;
c) l'élution de la rhGALC de la première résine chromatographique multimodale dans un premier tampon d'élution comprenant au moins 30 % de propylène glycol et/ou d'éthylène glycol (v/v) fournissant ainsi un premier éluat ;
d) le chargement du premier éluat sur une deuxième résine chromatographique multimodale comprenant un ligand anionique et hydrophobe ;
e) l'élution de la rhGALC de la deuxième résine chromatographique multimodale dans un deuxième tampon d'élution comprenant au moins 30 % de propylène glycol et/ou d'éthylène glycol (v/v) et ayant un pH inférieur à 5,5, fournissant ainsi un deuxième éluat ;
f) le chargement du deuxième éluat sur une troisième résine chromatographique ayant des ligands hydrophobes ; et
g) l'élution de la rhGALC de la troisième résine chromatographique comprenant un ligand comprenant un groupe éther, qui est sélectionnée parmi le groupe constitué par une résine de chromatographie multimodale, une résine d'échange d'anions, et une résine de chromatographie d'interaction hydrophobe (HIC) dans un tampon aqueux, fournissant ainsi un troisième éluat.

**7.** Procédé selon l'une quelconque des revendications précédentes, dans lequel la première résine chromatographique multimodale comprend comme ligand un composé de formule (I), (II) ou (III) :

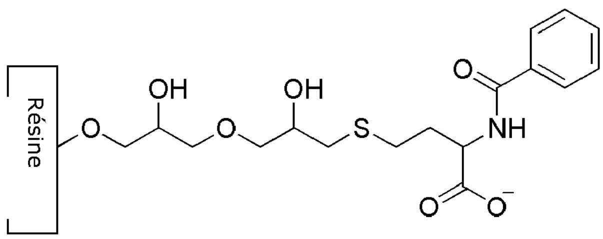

(I)

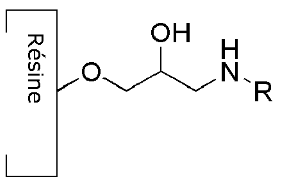

(II)

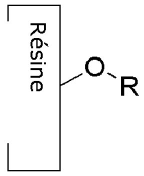

(III)

dans lequel le R des substances de formule (II) et (III) est un groupe fonctionnel de formule (IV) :

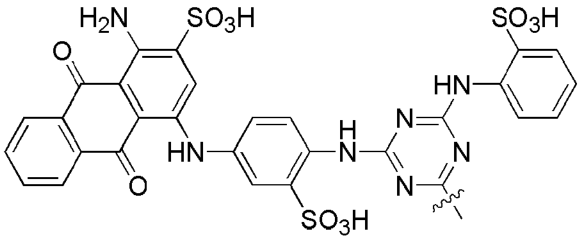

(IV).

**8.** Procédé selon l'une quelconque des revendications précédentes, dans lequel la première résine chromatographique est lavée dans un tampon de lavage comprenant au plus 20 % de propylène glycol et/ou d'éthylène glycol (v/v).

**9.** Procédé selon l'une quelconque des revendications précédentes, dans lequel le premier tampon d'élution comprend une concentration totale de propylène glycol et/ou d'ethylene glycol (v/v) de 40 à 60 %.

**10.** Procédé selon l'une quelconque des revendications précédentes, dans lequel la deuxième résine chromatographique multimodale comprend un ligand de formule (VIII) :

[RÉSINE]- 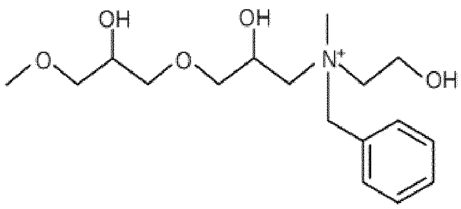

(VIII)

ou un ligand de formule (IX) :

[RÉSINE]- 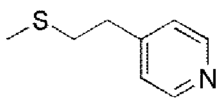

(IX).

**11.** Composition comprenant de la rhGALC, dans laquelle le rapport molaire entre la rhGALC de pleine longueur (80 kDa) et les principaux produits transformés (50 + 30 kDa) dans ladite composition est d'au moins 50/2,5.

**12.** Composition selon la revendication 11, qui peut être obtenue par le procédé de purification selon l'une quelconque des revendications précédentes.

**13.** Composition selon l'une quelconque des revendications 11 à 12 pour utilisation comme un médicament.

**14.** Composition selon l'une quelconque des revendications 11 à 12 pour utilisation dans le traitement de la leucodystrophie à cellules globoïdes (la maladie de Krabbe).

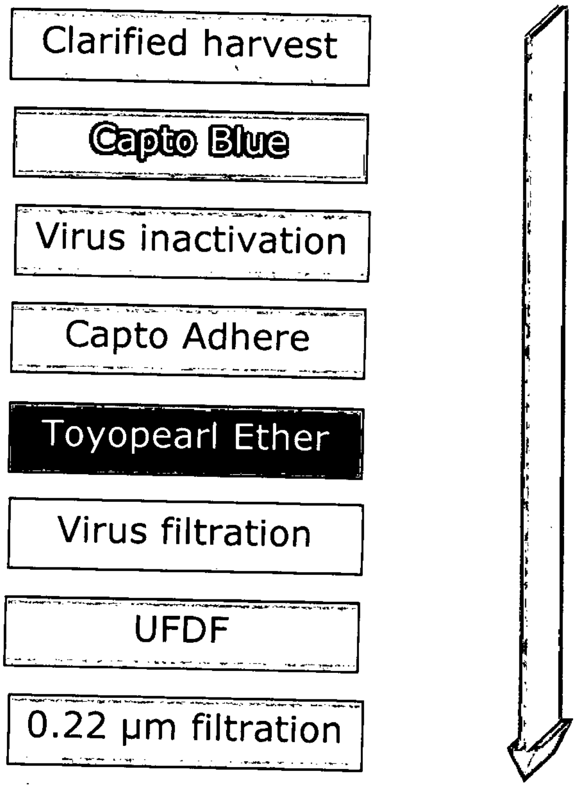
Clarified harvest
Capto Blue
Virus inactivation
Capto Adhere
Toyopearl Ether
Virus filtration
UFDF
0.22 µm filtration

Fig. 1

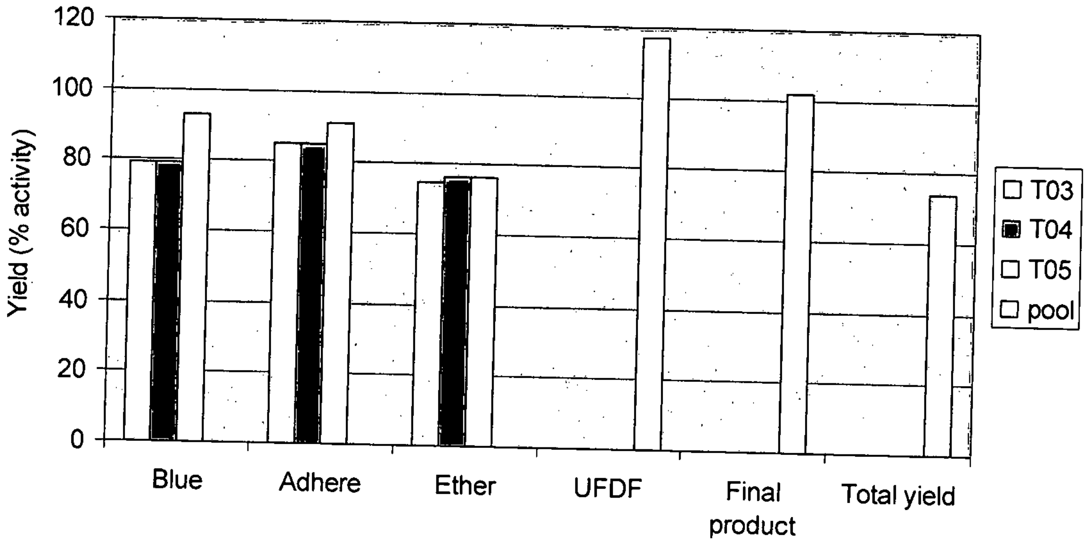
120
100
80
60
40
20
0
Yield (% activity)
Blue
Adhere
Ether
UFDF
Final product
Total yield
T03
T04
T05
pool

Fig. 2

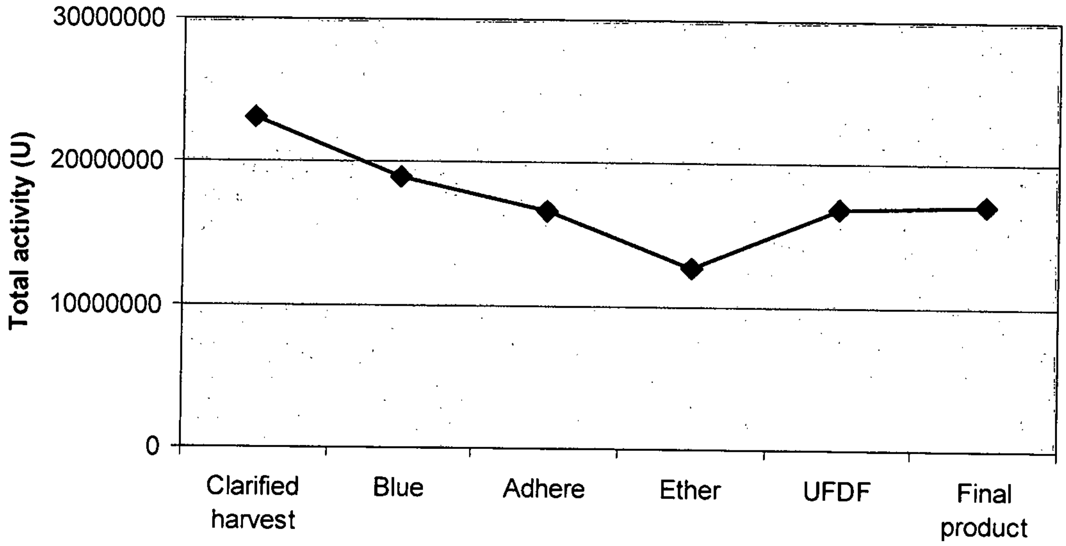
30000000
20000000
10000000
0
Total activity (U)
Clarified harvest
Blue
Adhere
Ether
UFDF
Final product

Fig. 3

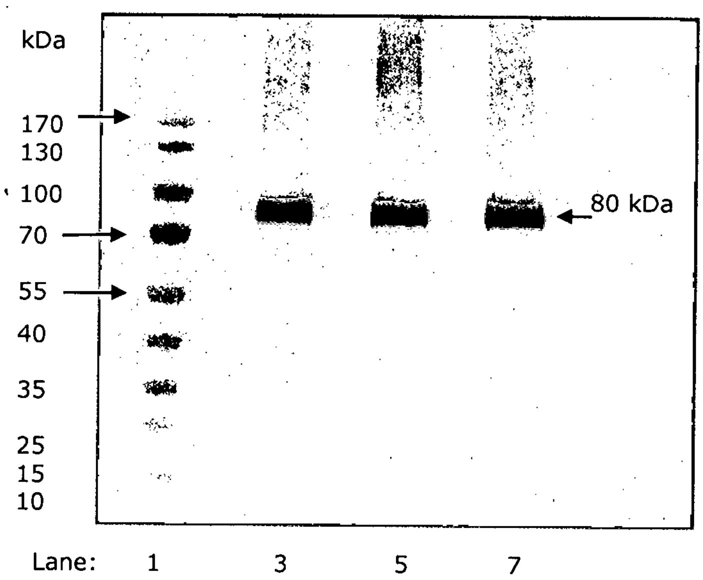


| Lane | Sample |
|---|---|
| 1. | PageRuler Prestained Protein Ladder (Fermentas) |
| 3. | StG02 |
| 5. | StG03 |
| 7. | TG1106 |

Fig. 4

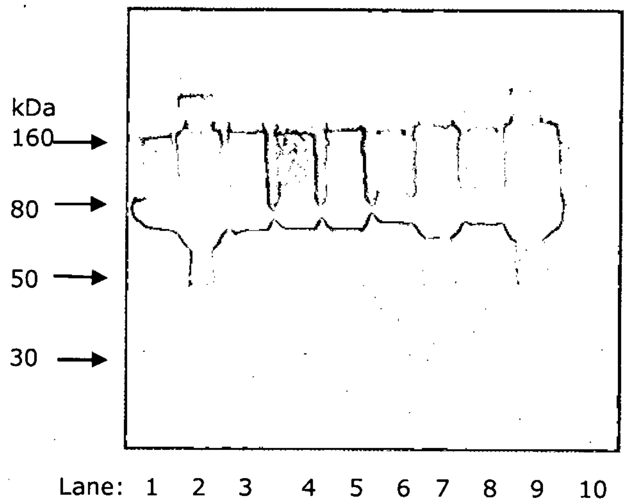


| Lane | Sample | |
|---|---|---|
| 1. | T01U | 3 µg |
| 2. | T01U | 34 µg |
| 3. | T05E | 7 µg |
| 4. | T04E | 6 µg |
| 5. | T03E | 6 µg |
| 6. | StG03 | 2 µg |
| 7. | StG03 | 18 µg |
| 8 | TG1106 | 3 µg |
| 9. | TG1106 | 32 µg |
| 10. | PageRuler Prestained Protein Ladder (Fermentas) | 5 µL |

Fig. 5

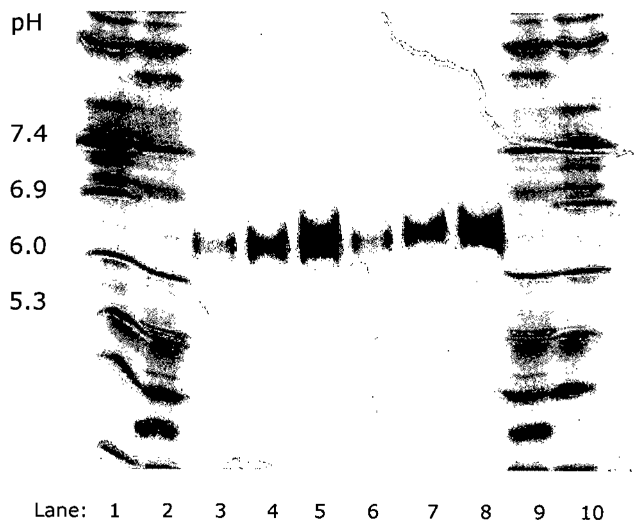


| Lane | Sample | Load |
|---|---|---|
| 1. | Sigma-Aldrich marker | |
| 2. | Serva IEF marker | |
| 3. | TG1106 | 1.6 µg |
| 4. | TG1106 | 3.2 µg |
| 5. | TG1106 | 6.3 µg |
| 6. | StG03 | 1.8 µg |
| 7. | StG03 | 3.6 µg |
| 8. | StG03 | 7.1 µg |
| 9. | Serva IEF marker | |
| 10. | Sigma-Aldrich marker | |

Fig. 6

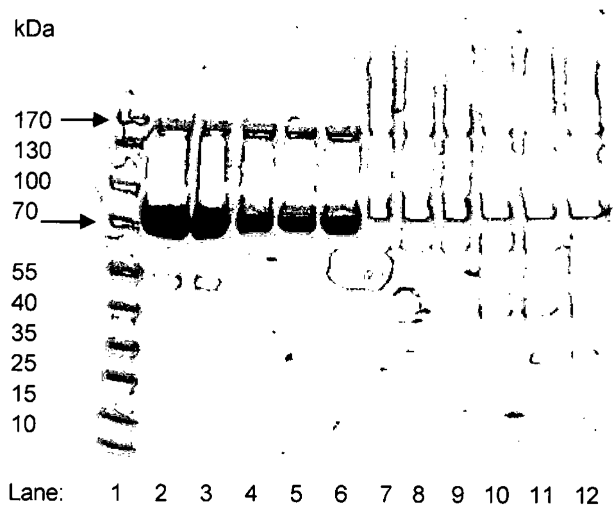


| Lane | Sample | Load |
|---|---|---|
| 1. | PageRuler Prestained Protein Ladder (Fermentas) | 5 µL |
| 2. | TG1106 | 16 µg |
| 3. | T01U | 11 µg |
| 4. | T05E | 7.3 µg |
| 5. | T04E | 6.2 µg |
| 6. | T03E | 6.1 µg |
| 7. | T05A | 2.9 µg |
| 8. | T04A | 2.9 µg |
| 9. | T03A | 2.7 µg |
| 10. | T05B | 3.6 µg |
| 11. | T04B | 3.5 µg |
| 12. | T03B | 3.9 µg |

Fig. 7

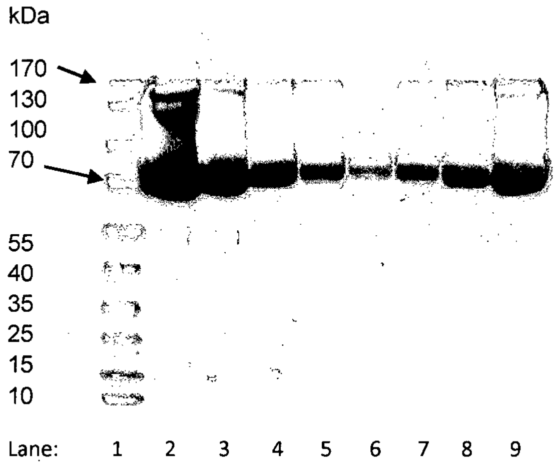


| Lane | Sample | Load |
|---|---|---|
| 1. | PageRuler Prestained Protein Ladder (Fermentas) | 5 µL |
| 2. | TG1106 | 41 µg |
| 3. | TG1106 | 20 µg |
| 4. | TG1106 | 10 µg |
| 5. | TG1106 | 5 µg |
| 6. | StG03 | 2 µg |
| 7. | StG03 | 5 µg |
| 8. | StG03 | 9 µg |
| 9. | StG03 | 18 µg |

Fig. 8

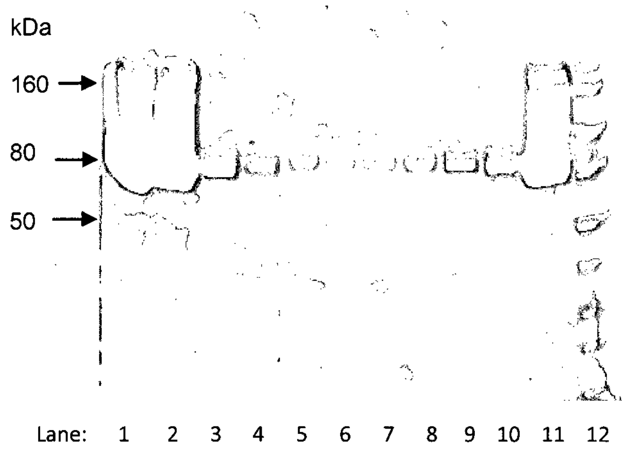


| Lane | Sample | Load |
|---|---|---|
| 1. | TG1106 | 32 µg |
| 2. | TG1106 | 16 µg |
| 3. | TG1106 | 3.2 µg |
| 4. | TG1106 | 0.32 µg |
| 5. | TG1106 | 0.16 µg |
| 6. | StG03 | 0.14 µg |
| 7. | StG03 | 0.18 µg |
| 8. | StG03 | 0.36 µg |
| 9. | StG03 | 0.9 µg |
| 10. | StG03 | 1.8 µg |
| 11. | StG03 | 18 µg |
| 12. | PageRuler Prestained Protein Ladder (Fermentas) | 5 µL |

Fig. 9

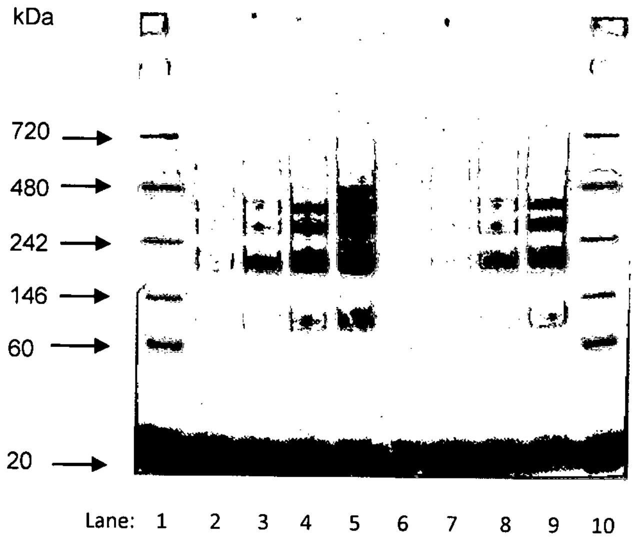


| Lane | Sample | Load |
|---|---|---|
| 1. | NativeMark unstained Protein standard | 5 µL |
| 2. | TG1106 | 5 µg |
| 3. | TG1106 | 10 µg |
| 4. | TG1106 | 19 µg |
| 5. | TG1106 | 38 µg |
| 6. | StG03 | 3 µg |
| 7. | StG03 | 5 µg |
| 8. | StG03 | 10 µg |
| 9. | StG03 | 21 µg |
| 10. | NativeMark unstained Protein standard | 5 µL |

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BEN-YOSEPH.** *Archives of Biochemistry and Biophysics,* 1979
- **SAKAI et al.** *J. Biochem.,* 1994
- **CHEN et al.** *Biochimica et Biophysica acta,* 1993

SEQUENCE LISTING

<110> ACE Biosciences A/S
Fogh, Jens
Andersson, Claes
Hydén, Pia
Gulstad, Pia Ringholm
Lundell, Kjersti
Hjertman, Magnus

<120> Production of galactocerebrosidase

<130> 50185dk01

<160> 2

<170> BiSSAP 1.2

<210> 1
<211> 3761
<212> DNA
<213> Homo sapiens

<220>
<221> source
<222> 1..3761
<223> /mol_type="unassigned DNA" /organism="Homo sapiens"

<400> 1

ggctactctc ggcttcctgg caacgccgag cgaaagctat gactgcggcc gcgggttcgg 60

cgggccgcgc cgcggtgccc ttgctgctgt gtgcgctgct ggcgcccggc ggcgcgtacg 120

tgctcgacga ctccgacggg ctgggccggg agttcgacgg catcggcgcg gtcagcggcg 180

gcggggcaac ctcccgactt ctagtaaatt acccagagcc ctatcgttct cagatattgg 240

attatctctt taagccgaat tttggtgcct ctttgcatat tttaaaagtg gaaataggtg 300

gtgatgggca gacaacagac ggcactgagc cctcccacat gcattatgca ctagatgaga 360

attatttccg aggatacgag tggtggttga tgaaagaagc taagaagagg aatcccaata 420

ttacactcat tgggttgcca tggtcattcc ctggatggct gggaaaaggt ttcgactggc 480

cttatgtcaa tcttcagctg actgcctatt atgtcgtgac ctggattgtg ggcgccaagc 540

gttaccatga tttggacatt gattatattg gaatttggaa tgagaggtca tataatgcca 600

attatattaa gatattaaga aaaatgctga attatcaagg tctccagcga gtgaaaatca 660

tagcaagtga taatctctgg gagtccatct ctgcatccat gctccttgat gccgaactct 720

tcaaggtggt tgatgttata ggggctcatt atcctggaac ccattcagca aaagatgcaa 780

agttgactgg gaagaagctt tggtcttctg aagactttag cactttaaat agtgacatgg 840

gtgcaggctg ctggggtcgc attttaaatc agaattatat caatggctat atgacttcca 900

caatcgcatg gaatttagtg gctagttact atgaacagtt gccttatggg agatgcgggt 960

tgatgacggc ccaagagcca tggagtgggc actacgtggt agaatctcct gtctgggtat 1020

cagctcatac cactcagttt actcaacctg gctggtatta cctgaagaca gttggccatt 1080
tagagaaagg aggaagctac gtagctctga ctgatggctt agggaacctc accatcatca 1140
ttgaaaccat gagtcataaa cattctaagt gcatacggcc atttcttcct tatttcaatg 1200
tgtcacaaca atttgccacc tttgttctta agggatcttt tagtgaaata ccagagctac 1260
aggtatggta taccaaactt ggaaaaacat ccgaaagatt tctttttaag cagctggatt 1320
ctctatggct ccttgacagc gatggcagtt tcacactgag cctgcatgaa gatgagctgt 1380
tcacactcac cactctcacc actggtcgca aaggcagcta cccgcttcct ccaaaatccc 1440
agcccttccc aagtacctat aaggatgatt tcaatgttga ttacccattt tttagtgaag 1500
ctccaaactt tgctgatcaa actggtgtat ttgaatattt tacaaatatt gaagaccctg 1560
gcgagcatca cttcacgcta cgccaagttc tcaaccagag acccattacg tgggctgccg 1620
atgcatccaa cacaatcagt attataggag actacaactg gaccaatctg actataaagt 1680
gtgatgttta catagagacc cctgacacag gaggtgtgtt cattgcagga agagtaaata 1740
aaggtggtat tttgattaga agtgccagag gaattttctt ctggattttt gcaaatggat 1800
cttacagggt tacaggtgat ttagctggat ggattatata tgctttagga cgtgttgaag 1860
ttacagcaaa aaaatggtat acactcacgt taactattaa gggtcatttc gcctctggca 1920
tgctgaatga caagtctctg tggacagaca tccctgtgaa ttttccaaag aatggctggg 1980
ctgcaattgg aactcactcc tttgaatttg cacagtttga caactttctt gtggaagcca 2040
cacgctaata cttaacaggg catcatagaa tactctggat tttcttccct tctttttggt 2100
tttggttcag agccaattct tgtttcattg gaacagtata tgaggctttt gagactaaaa 2160
ataatgaaga gtaaaagggg agagaaattt atttttaatt taccctgtgg aagattttat 2220
tagaattaat tccaagggga aaactggtga atctttaaca ttacctggtg tgttccctaa 2280
cattcaaact gtgcattggc catacccta ggagtggttt gagtagtaca gacctcgaag 2340
ccttgctgct aacactgagg tagctctctt catcttattt gcaagcggtc ctgtagatgg 2400
cagtaacttg atcatcactg agatgtattt atgcatgctg accgtgtgtc caagtgagcc 2460
agtgtcttca tcacaagatg atgctgccat aatagaaagc tgaagaacac tagaagtagc 2520
tttttgaaaa ccacttcaac ctgttatgct ttatgctcta aaaagtattt ttttattttc 2580
ctttttaaga tgatactttt gaaatgcagg atatgatgag tgggatgatt ttaaaaacgc 2640
ctctttaata aactacctct aacactattt ctgcggtaat agatattagc agattaattg 2700
ggttatttgc attatttaat tttttgatt ccaagttttg gtcttgtaac cactataact 2760
ctctgtgaac gtttttccag gtggctggaa gaaggaagaa aacctgatat agccaatgct 2820
gttgtagtcg tttcctcagc ctcatctcac tgtgctgtgg tctgtcctca catgtgcact 2880

ggtaacagac tcacacagct gatgaatgct tttctctcct tatgtgtgga aggaggggag 2940
cacttagaca tttgctaact cccagaattg gatcatctcc taagatgtac ttacttttta 3000
aagtccaaat atgtttatat ttaaatatac gtgagcatgt tcatcatgtt gtatgattta 3060
tactaagcat taatgtggct ctatgtagca aatcagttat tcatgtaggt aaagtaaatc 3120
tagaattatt tataagaatt actcattgaa ctaattctac tatttaggaa tttataagag 3180
tctaacatag gcttagctac agtgaagttt tgcattgctt ttgaagacaa gaaaagtgct 3240
agaataaata agattacaga gaaaattttt tgttaaaacc aagtgatttc cagctgatgt 3300
atctaatatt ttttaaaaca aacattatag aggtgtaatt tatttacaat aaaatgttcc 3360
tactttaaat atacaattca gtgagttttg ataaattgat atacccatgt aaccaacact 3420
ccagtcaagc ttcagaatat ttccatcacc ccagaaggtt ctcttgtata cctgctcagt 3480
cagttccttt cactcccaat tgttggcagc cattgatagg aattctatca ctataggtta 3540
gttttctttg ttccagaaca tcatgaaagc ggcgtcatgt actgtgtatt cttatgaatg 3600
gtttctttcc atcagcataa tgatttgaga ttggtccatg ttgtgtgatt cagtggtttg 3660
ttccttctta tttctgaaga gttttccatt gtatgaatat accacaattt gtttcctccc 3720
caccagtttc tgatactaca attaaaactg tctacattta c 3761

<210> 2
<211> 669
<212> PRT
<213> Homo sapiens

<400> 2

```
Met Thr Ala Ala Ala Gly Ser Ala Gly Arg Ala Ala Val Pro Leu Leu
1               5                   10                  15
Leu Cys Ala Leu Leu Ala Pro Gly Gly Ala Tyr Val Leu Asp Asp Ser
            20                  25                  30
Asp Gly Leu Gly Arg Glu Phe Asp Gly Ile Gly Ala Val Ser Gly Gly
        35                  40                  45
Gly Ala Thr Ser Arg Leu Leu Val Asn Tyr Pro Glu Pro Tyr Arg Ser
    50                  55                  60
Gln Ile Leu Asp Tyr Leu Phe Lys Pro Asn Phe Gly Ala Ser Leu His
65                  70                  75                  80
Ile Leu Lys Val Glu Ile Gly Gly Asp Gly Gln Thr Thr Asp Gly Thr
                85                  90                  95
Glu Pro Ser His Met His Tyr Ala Leu Asp Glu Asn Tyr Phe Arg Gly
            100                 105                 110
Tyr Glu Trp Trp Leu Met Lys Glu Ala Lys Lys Arg Asn Pro Asn Ile
        115                 120                 125
Thr Leu Ile Gly Leu Pro Trp Ser Phe Pro Gly Trp Leu Gly Lys Gly
    130                 135                 140
Phe Asp Trp Pro Tyr Val Asn Leu Gln Leu Thr Ala Tyr Tyr Val Val
145                 150                 155                 160
Thr Trp Ile Val Gly Ala Lys Arg Tyr His Asp Leu Asp Ile Asp Tyr
                165                 170                 175
Ile Gly Ile Trp Asn Glu Arg Ser Tyr Asn Ala Asn Tyr Ile Lys Ile
            180                 185                 190
Leu Arg Lys Met Leu Asn Tyr Gln Gly Leu Gln Arg Val Lys Ile Ile
```

```
        195                 200                 205
Ala Ser Asp Asn Leu Trp Glu Ser Ile Ser Ala Ser Met Leu Leu Asp
    210                 215                 220
Ala Glu Leu Phe Lys Val Val Asp Val Ile Gly Ala His Tyr Pro Gly
225                 230                 235                 240
Thr His Ser Ala Lys Asp Ala Lys Leu Thr Gly Lys Lys Leu Trp Ser
                245                 250                 255
Ser Glu Asp Phe Ser Thr Leu Asn Ser Asp Met Gly Ala Gly Cys Trp
            260                 265                 270
Gly Arg Ile Leu Asn Gln Asn Tyr Ile Asn Gly Tyr Met Thr Ser Thr
        275                 280                 285
Ile Ala Trp Asn Leu Val Ala Ser Tyr Tyr Glu Gln Leu Pro Tyr Gly
    290                 295                 300
Arg Cys Gly Leu Met Thr Ala Gln Glu Pro Trp Ser Gly His Tyr Val
305                 310                 315                 320
Val Glu Ser Pro Val Trp Val Ser Ala His Thr Thr Gln Phe Thr Gln
                325                 330                 335
Pro Gly Trp Tyr Tyr Leu Lys Thr Val Gly His Leu Glu Lys Gly Gly
            340                 345                 350
Ser Tyr Val Ala Leu Thr Asp Gly Leu Gly Asn Leu Thr Ile Ile Ile
        355                 360                 365
Glu Thr Met Ser His Lys His Ser Lys Cys Ile Arg Pro Phe Leu Pro
    370                 375                 380
Tyr Phe Asn Val Ser Gln Gln Phe Ala Thr Phe Val Leu Lys Gly Ser
385                 390                 395                 400
Phe Ser Glu Ile Pro Glu Leu Gln Val Trp Tyr Thr Lys Leu Gly Lys
                405                 410                 415
Thr Ser Glu Arg Phe Leu Phe Lys Gln Leu Asp Ser Leu Trp Leu Leu
            420                 425                 430
Asp Ser Asp Gly Ser Phe Thr Leu Ser Leu His Glu Asp Glu Leu Phe
        435                 440                 445
Thr Leu Thr Thr Leu Thr Thr Gly Arg Lys Gly Ser Tyr Pro Leu Pro
    450                 455                 460
Pro Lys Ser Gln Pro Phe Pro Ser Thr Tyr Lys Asp Asp Phe Asn Val
465                 470                 475                 480
Asp Tyr Pro Phe Phe Ser Glu Ala Pro Asn Phe Ala Asp Gln Thr Gly
                485                 490                 495
Val Phe Glu Tyr Phe Thr Asn Ile Glu Asp Pro Gly Glu His His Phe
            500                 505                 510
Thr Leu Arg Gln Val Leu Asn Gln Arg Pro Ile Thr Trp Ala Ala Asp
        515                 520                 525
Ala Ser Asn Thr Ile Ser Ile Ile Gly Asp Tyr Asn Trp Thr Asn Leu
    530                 535                 540
Thr Ile Lys Cys Asp Val Tyr Ile Glu Thr Pro Asp Thr Gly Gly Val
545                 550                 555                 560
Phe Ile Ala Gly Arg Val Asn Lys Gly Gly Ile Leu Ile Arg Ser Ala
                565                 570                 575
Arg Gly Ile Phe Phe Trp Ile Phe Ala Asn Gly Ser Tyr Arg Val Thr
            580                 585                 590
Gly Asp Leu Ala Gly Trp Ile Ile Tyr Ala Leu Gly Arg Val Glu Val
        595                 600                 605
Thr Ala Lys Lys Trp Tyr Thr Leu Thr Leu Thr Ile Lys Gly His Phe
    610                 615                 620
Ala Ser Gly Met Leu Asn Asp Lys Ser Leu Trp Thr Asp Ile Pro Val
625                 630                 635                 640
Asn Phe Pro Lys Asn Gly Trp Ala Ala Ile Gly Thr His Ser Phe Glu
                645                 650                 655
Phe Ala Gln Phe Asp Asn Phe Leu Val Glu Ala Thr Arg
            660                 665
```